(19) Europäisches Patentamt
European Patent Office
Office européen des brevets

(11) Veröffentlichungsnummer: **0 115 080**
**B1**

# (12) EUROPÄISCHE PATENTSCHRIFT

(45) Veröffentlichungstag der Patentschrift: **16.04.86**

(51) Int. Cl.⁴: **C 07 C 93/00**, A 61 K 31/13

(21) Anmeldenummer: **83113176.8**

(22) Anmeldetag: **28.12.83**

(54) **(Omega-(Diäthylamino)-alkoxy)-alpha-(äthyl)-benzhydrolderivate, ihre Säureadditionssalze und quaternären Salze, Verfahren zu ihrer Herstellung und diese Verbindungen enthaltende Arzneimittel.**

(30) Priorität: **28.12.82 HU 418282**

(43) Veröffentlichungstag der Anmeldung: **08.08.84 Patentblatt 84/32**

(45) Bekanntmachung des Hinweises auf die Patenterteilung: **16.04.86 Patentblatt 86/16**

(84) Benannte Vertragsstaaten: **AT BE CH DE FR GB IT LI LU NL SE**

(56) Entgegenhaltungen:
**GB-A- 1 478 185**
**US-A- 3 075 014**

(73) Patentinhaber: **RICHTER GEDEON VEGYESZETI GYAR R.T.**
**Gyömröi ut 19-21**
**H-1475 Budapest X (HU)**

(72) Erfinder: **Tòth, Edit, Dipl.-Ing.**
**Szabolcska u. 7**
**H-1114 Budapest (HU)**
Erfinder: **Törley, Jòzsef, Dipl.-Ing.**
**Katona J. u. 41**
**H-1137 Budapest (HU)**
Erfinder: **Fekete, György, Dr.**
**Széher u. 62**
**H-1021 Budapest (HU)**
Erfinder: **Szporny, Lászlò, Dr.**
**Szabolcska u. 7**
**H-1114 Budapest (HU)**
Erfinder: **Vereczkey, Lászlò, Dr.**
**Lajos u. 109**
**H-1036 Budapest (HU)**
Erfinder: **Pálosi, Eva, Dr.**
**Vend u. 21**
**H-1025 Budapest (HU)**
Erfinder: **Klebovich, Imre, Dr.**
**Karvaly u. 4**
**H-1125 Budapest (HU)**
Erfinder: **Vittay, Pál, Dr.**
**Jòzsef krt. 14**
**H-1085 Budapest (HU)**
Erfinder: **Görög, Sándor, Dr. Dipl.-Chem.**
**Vajda P. u. 43**
**H-1089 Budapest (HU)**
Erfinder: **Hajdu, István, Dipl.-Chem.**
**Tátra tér B/4**
**H-1205 Budapest (HU)**

(74) Vertreter: **Beszédes, Stephan G. Dr.**
**Münchener Strasse 80a Postfach 1168**
**D-8060 Dachau/DE**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 080 B1

Jouve, 18, rue St-Denis, 75001 Paris, France

**Beschreibung**

Die Erfindung betrifft neue [ω-(Diäthylamino)-alkoxy]-α-[äthyl]-benzhydrolderivate, ihre Säureadditionssalze und quaternären Salze, ein Verfahren zu ihrer Herstellung und diese Verbindungen enthaltende Arzneimittel.

Gegenstand der Erfindung sind [ω-(Diäthylamino)-alkoxy]-α[äthyl]-benzhydrolderivate der allgemeinen Formel

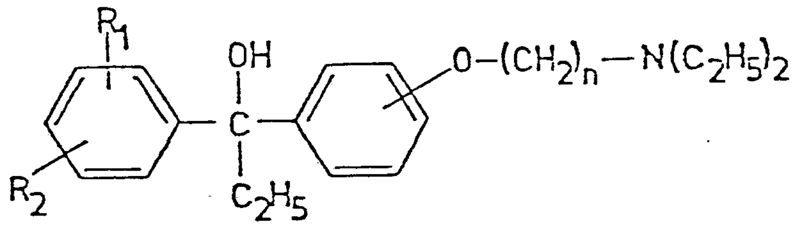

(I)

worin

$R_1$ für ein Wasserstoffatom, ein Halogenatom, einen Trihalogenmethylrest oder einen geradkettigen oder verzweigten Alkyl- oder Alkoxyrest mit je 1 bis 4 Kohlenstoffatom(en) steht,

$R_2$ ein Halogenatom, einen Trihalogenmethylrest oder einen Alkyl- oder Alkoxyrest mit je 1 bis 4 Kohlenstoffatom(en) bedeutet und

n eine ganze Zahl von 1 bis 4 ist,

sowie ihre Säureadditionssalze und quaternären Salze.

Die Herstellung von Verbindungen ähnlicher Struktur ist in den folgenden Literaturstellen beschrieben : C.A. *22,* 410[1] ; *35,* 1781[2] ; *40,* 4712[5] ; *42,* P 1015 b ; *47,* 9548 e ; *50,* 12390 c ; *50,* 2509 i ; *55,* 17915 e ; *55,* 15413 b ; *75,* P 103682 b , *76,* P 119921 k ; *82,* 16477 q ; *90,* 86062 q ; *92,* 52927 b. Es wird jedoch nirgends erwähnt, daß die hergestellten Verbindungen eine pharmakologische Wirkung haben.

Ferner sind aus der britischen Patentschrift 1 478 185 gemäß deren Patentanspruch 1 substituierte Benzhydrolderivate, bei welchen der eine Benzolring unter anderen durch 1 oder mehr Halogenatom(e), Trihalogenmethylrest(e) und/oder geradkettige [n] und/oder verzweigte [n] gesättigte [n] und/oder ungesättigte [n] aliphatische [n] Kohlenwasserstoffrest(e) mit 1 bis 6 Kohlenstoffatom(en) substituiert sein kann, sowie ferner aus ihren Beispielen 13 und 14 4-(β-Diäthyllaminoäthoxy)-α-(äthyl)-benzhydrol und 4-(β-Diäthylaminoäthoxy)-α-(äthyl)-benzhydroläthobromid bekannt. Als pharmakologische Wirksamkeit dieser Verbindungen ist die Blockier- oder Induzierwirkung auf das Fremdsubstanzen umsetzende mikrosomale Enzymsystem angegeben. Für Substanzen, deren Inaktivierung durch die enzyminduzierende Wirkung der Verbindungen der britischen Patentschrift 1 478 185 beschleunigt wird, sind Bilirubin, Insektizide, Progesteron, Hexobarbital, Meprobamat und Bromsulfophthalein speziell erörtert.

Zwar können die Verbindungen der britischen Patentschrift 1 478 185 gemäß deren Patentanspruch 1 am einen Benzolring 1 oder mehr der Substituenten, welche $R_2$ in der Formel I der erfindungsgemäßen Verbindungen bedeuten kann, aufweisen, die erfindungsgemäßen Verbindungen bedeuten kann, aufweisen, die erfindungsgemäßen Verbindungen sind jedoch im Gegensatz zu den Verbindungen der genannten Druckschrift, welche am zweiten Benzolring nicht substituiert sind, am zweiten Benzolring zwingend durch ein [ω-(Diäthylamino)-alkoxy]-rest substituiert.

Zwar haben die Verbindungen der Beispiele 13 und 14 der britischen Patentschrift 1 478 185, welche nicht unter deren Patentanspruch 1 fallen, einen β-Diäthylaminoäthoxysubstituenten, welcher mit dem für den Fall, daß n in der Formel I der erfindungsgemäßen Verbindungen 2 ist, in den erfindungsgemäßen Verbindungen vorliegenden Diäthylaminoäthoxysubstituenten identisch ist, an ihrem einen Benzolring, auch von diesen bekannten Verbindungen, deren anderer Benzolring nicht substituiert ist, unterscheiden sich aber alle erfindungsgemäßen Verbindungen darin, daß auch ihr anderer Benzolring substituiert ist.

Weiterhin sind in der US-Patentschrift 3 075 014 substituierte Diphenylbutanole beziehungsweisebutenole und entsprechende höhere Alkanole beziehungsweise Alkenole beschrieben, wobei als niedrigste spezielle Alkanole Pentanole genannt sind. Als spezielle Indikationen ihrer pharmakologischen Wirkung sind die Behandlung von Hypercholesterinämie und Herzgefäßerkrankungen, welche durch hohe Cholesterinblutspiegel verschärft werden, sowie entzündungshemmende, östrogene und antiöstrogene Wirkungen angegeben.

Die erfindungsgemäßen Verbindungen unterscheiden sich von allen Verbindungen der US-Patentschrift 3 075 014 darin, daß die letzteren mindestens Butanole sind, während die erfindungsgemäßen Verbindungen zwingend Propanole sind.

Vorzugsweise ist, beziehungsweise sind, das, beziehungsweise die, Halogenatom(e), für das, beziehungsweise die, $R_1$ und/oder $R_2$ stehen kann, beziehungsweise können, [ein] Chlor-, Fluor- und/oder Bromatom(e), insbesondere [ein] Chlor- und/oder Fluoratom(e).

Es ist auch bevorzugt, daß der, beziehungsweise die, Tri-halogenmethylrest(e), für den, beziehungs-

weise die, $R_1$ und/oder $R_2$ stehen kann, beziehungsweise können , [ein] Trifluormethylrest(e) und/oder Trichlormethylrest(e), insbesondere der, beziehungsweise die, erstere(n), ist, beziehungsweise sind.

Ferner ist es bevorzugt, daß der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind.

Weiterhin ist es bevorzugt, daß die Alkylreste, für die $R_3$ und $R_4$ stehen, solche mit 3 oder 4, insbesondere 3, Kohlenstoffatomen sind.

Außerdem ist es bevorzugt, daß n 2 bis 4, insbesondere 2 oder 3, ist.

Bevorzugt ist der substituierte [ω-(Diäthylamino)-alkoxy]-rest in der 4-Stellung des Benzolringes, an welchem er hängt.

Besonders bevorzugte erfindungsgemäße [ω-(Diäthylamino)-alkoxy]-α-[äthyl]-benzhydrolderivate sind 3-Trifluormethyl-4'-[3-(diäthylamino)-propoxy]-α-äthyl-benzhydrol, 2-Methoxy-4'-[2-(diäthylamino)-äthoxy]-α-äthyl-benzhydrol, 4-Trifluormethyl-4'-[2-(diäthylamino)-äthoxy]-α-äthylbenzhydrol, 3-Chlor-4'-[3-(diäthylamino)-propoxy]-α-äthyl-benzhydrol, 3-Chlor-4'-[2-(diäthylamino)-äthoxy]-α-äthyl-benzhydrol, 4-Fluor-4'-[3-(diäthylamino)-propoxy]-α-äthyl-benzhydrol, 2-Methoxy-4'-[3-(diäthylamino)-propoxy]-α-äthyl-benzhydrol, 2,5-Dimethyl-4'-[2-(diäthylamino)-äthoxy]-α-äthyl-benzhydrol, 4-Chlor-4'-[3-(diäthylamino)-propoxy]-α-äthyl-benzhydrol, 3-Trifluormethyl-4'-[2-(diäthylamino)-äthoxy]-α-äthyl-benzhydrol und 4-Chlor-4'-[2-(diäthylamino)-äthoxy]-α-äthyl-benzhydrol sowie ihre Salze.

Gegenstand der Erfindung ist auch ein Verfahren zur Herstellung der erfindungsgemäßen Verbindungen, welches dadurch gekennzeichnet ist, daß

a) am Benzolring ω-(diäthylamino)-alkoxysubstituierte Propiophenone der allgemeinen Formel

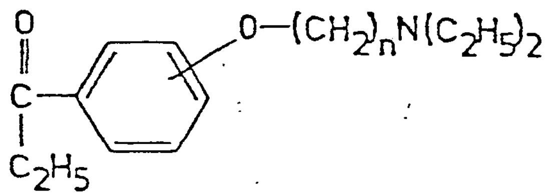

(II)

worin n wie oben festgelegt ist, mit eine Phenylgruppe aufweisenden metallorganischen Verbindungen der allgemeinen Formel

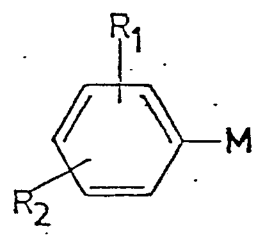

(III)

worin

$R_1$ und $R_2$ wie oben festgelegt ist und

M für ein Alkalimetallatom, vorzugsweise Lithium-, Natrium- oder Kaliumatom oder einen Rest der Formel MgX mit X = Halogenatom steht,

umgesetzt werden oder

b) [ω-(Halogen)-alkoxy]-α-[äthyl]-benzhydrolderivate der allgemeinen Formel

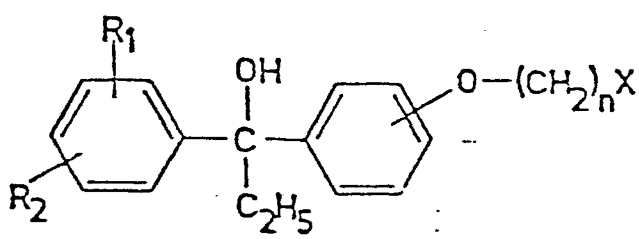

(IV)

worin

$R_1$, $R_2$ und n wie oben festgelegt sind und

X für ein Halogenatom steht,

mit Diäthylamin umgesetzt werden oder

c) [ω-(Diäthylamino)-alkoxy]-benzophenonderivate der allgemeinen Formel

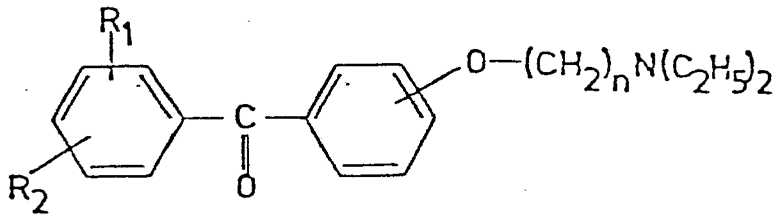

(V)

worin $R_1$, $R_2$ und n wie oben festgelegt sind, mit eine Äthylgruppe aufweisenden metallorganischen Verbindungen, insbesondere Äthylmagnesiumhalogeniden oder Äthyllithium, umgesetzt werden oder

d) am Benzolring substituierte Propiophenone der allgemeinen Formel

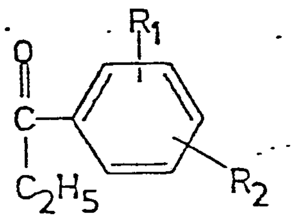

(VI)

worin $R_1$ und $R_2$ wie oben festgelegt sind, mit eine {[ω-(Diäthylamino)-alkoxy]-phenyl}-gruppe aufweisenden Grignard-Verbindungen der allgemeinen Formel

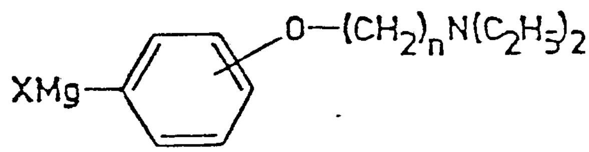

(VII)

worin die Bedeutung von

n wie oben festgelegt ist und

X für ein Halogenatom steht,

umgesetzt werden oder

e) [Hydroxy]-α-[äthyl]-benzhydrolderivate der allgemeinen Formel

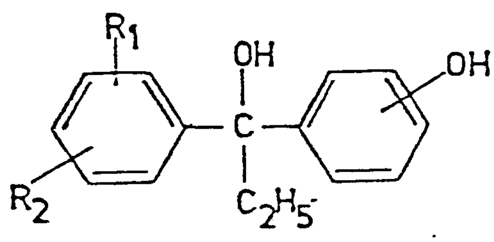

(VIII)

worin $R_1$ und $R_2$ wie oben festgelegt sind, vorzugsweise in Form ihrer Alkaliphenolate oder quaternären Ammoniumphenolate, mit tertiären Aminen der allgemeinen Formel

$$(C_2H_5)_2N{-}(CH_2)_n{-}X \qquad (IX)$$

worin

X für einen Alkylsulfonyloxy- oder Arylsulfonyloxyrest oder ein Halogenatom steht und

n wie oben festgelegt ist, oder mit deren Salzen, vorzugsweise in Gegenwart von säurebindenden Mitteln,

umgesetzt werden oder

f) [ω-(Diäthylamino)-alkoxy]-α-[äthinyl]-beziehungsweise [ω-(Diäthylamino)-alkoxy]-α-[vinyl]-benzhydrole der allgemeinen Formel

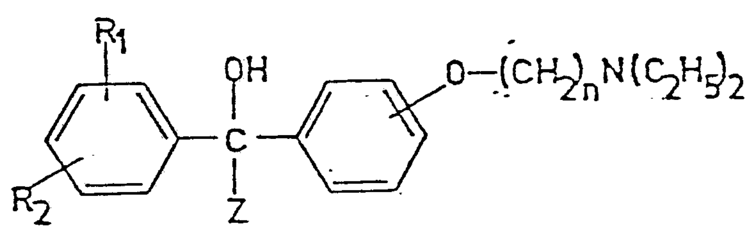

(X)

worin

$R_1$, $R_3$ und n wie oben festgelegt sind und

Z für einen Äthinyl- oder Vinylrest steht,

reduziert werden, worauf gegebenenfalls in an sich bekannter Weise die erhaltenen [ω-(Diäthylamino)-alkoxy]-α-[äthyl]-benzhydrolderivate der allgemeinen Formel I mit Säuren in Säureadditionssalze oder mit Quaternisierungsmitteln in quaternäre Salze überführt werden oder gegebenenfalls die erhaltenen Säureadditionssalze der [ω-(Diäthylamino)-alkoxy]-α-[äthyl]-benzhydrolderivate der allgemeinen Formel I in die entsprechenden freien Basen der allgemeinen Formel I und/oder in andere Säureadditionssalze überführt werden.

Die Ausgangsstoffe sind bekannt beziehungsweise können mittels literaturbekannter Verfahren hergestellt werden. Die Ketone der allgemeinen Formeln (II), (V) und (VI) können zum Beispiel mit der Ketonsynthese von Friedel-Crafts erhalten werden (G.A. Olah : Friedel-Crafts and related reactions, Bd. III/1, Ed. : Interscience Publishers 1964, S. 1-63).

Die Verbindungen der allgemeinen Formeln (III) und (VII) können zum Beispiel hergestellt werden, indem man aus den entsprechenden Arylhalogeniden in an sich bekannter Weise das Grignard-Reagens bildet (M.S. Kharash et al. : Grignard reactions of nonmetallic substances, Ed. Prentice-Hall. Inc., 1954, S. 5-90). Alkalimetallorganische Verbindungen sind zum Beispiel nach Houben-Weyl : Methoden der organischen Chemie, Bd. XIII/1, S. 134-159 sowie 389-405 (1970) zugänglich.

Die Verbindungen der allgemeinen Formeln (IV) und (VIII) können zum Beispiel aus den entsprechenden Propiophenonen synthetisiert werden, indem man diese auf literaturbekannte Weise mit Grignard-Reagentien umsetzt (z. B. M.S. Kharash et al. : Grignard reactions of nonmetallic substances, Ed. Prentice-Hall In., 1954, S. 138-143).

Die Ausgangsstoffe der allgemeinen Formel (X) erhält man zum Beispiel, indem man — wenn Z für einen Vinylrest steht — die Benzophenone der allgemeinen Formel (VI) mit einem Vinylmagnesiumhalogenid umsetzt, beziehungsweise — wenn Z für einen Äthinylrest steht — die Benzophenone der allgemeinen Formel (V) zum Beispiel auf die in der ungarischen Patentschrift Nr. 166 769 beschriebene Weise äthinyliert.

Gemäß einer bevorzugten Ausführungsform der Verfahrensvariante a) wird das Propiophenon der allgemeinen Formel (II) in einem wasserfreien inerten organischen Lösungsmittel mit der metallorganischen Verbindung der allgemeinen Formel (III), vorzugsweise dem entsprechend substituierten Phenylmagnesiumchlorid oder -bromid, oder dem entsprechend substituierten Phenyllithium, umgesetzt. Als Lösungsmittel werden aprotische Lösungsmittel, zum Beispiel aliphatische Äther wie Diäthyläther, Di-n-butyläther, Diäthylenglykoldimethyläther, alicyclische Äther, wie Tetrahydrofuran und Dioxan, aliphatische oder aromatische Kohlenwasserstoffe, zum Beispiel Ligroin, Benzol, Toluol, Xylol, ferner Dimethylsulfoxyd, Hexamethylphosphoramid oder Gemische der aufgeführten Lösungsmittel verwendet. Die metallorganische Verbindung wird in wenigstens der äquimolaren Menge eingesetzt. Die Umsetzung wird bevorzugt unter Schutzgasatmosphäre, zum Beispiel unter Stickstoff oder Argon, ausgeführt. Die Reaktionstemperatur liegt zwischen —60 °C und dem Siedepunkt des Lösungsmittels und beträgt vorzugsweise —30 bis 100 °C.

Nach Ablauf der Reaktion wird die metallorganische Verbindung — vorzugsweise mit einer wäßrigen Ammoniumchloridlösung — zersetzt und die Verbindung der allgemeinen Formel (I) abgetrennt. Das Produkt kann in an sich bekannter Weise, zum Beispiel durch Destillation oder Umkristallisation, gereinigt werden.

Gemäß der Verfahrensvariante b) werden Verbindungen der allgemeinen Formel (IV), worin X vorzugsweise für Chlor oder Brom steht, mit Diäthylamin umgesetzt. Die Umsetzung erfolgt vorzugsweise in einem organischen Lösungsmittel und in Gegenwart eines Säureakzeptors. Als Lösungsmittel sind zum Beispiel Kohlenwasserstoffe, wie Ligroin, Benzol oder Toluol, ferner chlorierte Kohlenwasserstoffe, zum Beispiel Chloroform, sowie Äther, wie Dioxan, oder Alkohole, zum Beispiel Äthanol, Ester, zum Beispiel Äthylacetat, Säureamide, zum Beispiel Dimethylformamid, Ketone, wie Aceton oder Methylisobutylketon, oder aber Gemische der aufgeführten Lösungsmittel geeignet. Als Säureakzeptor verwendet man zweckmäßig anorganische oder tertiäre organische Basen, insbesondere Amine, oder aber den Überschuß des Diäthylamins. Wenn man zum Binden des entstehenden Halogenwasserstoffes einen Überschuß an Diäthylamin oder eine tertiäre organische Base verwendet, können diese gleichzeitig als Lösungsmittel wirken. Die Umsetzung nimmt man bei einer Temperatur zwischen 20 °C und dem Siedepunkt des verwendeten Lösungsmittels vor. Nach Beendigung der Reaktion wird das Produkt isoliert. Zu diesem Zweck kann das Reaktionsgemisch zum Beispiel in Wasser gegossen und das Produkt mit einem organischen Lösungsmittel extrahiert werden. Die organische Phase wird mit Wasser halogenidfrei gewaschen, getrocknet und dann eingedampft. Das rohe Produkt kann zum Beispiel durch Destillation oder Umkristallisation gereinigt werden.

Gemäß der Verfahrensvariante c) wird das Benzophenon der allgemeinen Formel (V) mit der wenigstens äquivalenten Menge von vorzugsweise Äthylmagnesiumbromid oder Äthylmagnesiumjodid oder Äthyllithium zur Reaktion gebracht. Die Umsetzung wird ähnlich wie die Verfahrensvariante a) in einem inerten, wasserfreien organischen Lösungsmittel vorgenommen.

Gemäß der Verfahrensvariante d) werden Grignard-Verbindungen der allgemeinen Formel (VII), insbesondere solche, in denen X für Brom steht, in einem wasserfreien, inerten organischen Lösungsmittel mit wenigstens der äquivalenten Menge des Propiophenons der allgemeinen Formel (VI) umgesetzt.

Gemäß einer bevorzugten Ausführungsform der Verfahrensvariante e) wird die Verbindung der allgemeinen Formel (VIII), vorzugsweise in Form ihres Alkalisalzes oder quaternären Ammoniumsalzes, mit dem tertiären Amin der allgemeinen Formel (IX) kondensiert. Als tertiäre Amine kommen zum Beispiel Diäthylaminoalkylmesylat, -tosylat, -bromid und insbesondere -chlorid in Frage ; das Amin wird entweder in Form der freien Base oder als Salz, zum Beispiel als Hydrohalogenid, eingesetzt. Die Umsetzung wird vorzugsweise in einem inerten Lösungsmittel und in Gegenwart eines Säureakzeptors vorgenommen. Das Lösungsmittel kann gegebenenfalls Wasser enthalten. Als Lösungsmittel kommen zum Beispiel in Frage : Ester, wie Äthylacetat ; Äther, wie Dioxan, Tetrahydrofuran oder Diäthyläther ; Kohlenwasserstoffe, wie Ligroin, Benzol, Toluol oder Xylol ; halogenierte Kohlenwasserstoffe, wie Chloroform oder Chlorbenzol ; Säureamide, wie Dimethylformamid ; Ketone, wie Aceton, Methyläthylketon oder Methylisobutylketon ; Alkohole, wie Äthanol oder Propanol. Aus den Verbindungen der allgemeinen Formel (VIII) können die Phenolate in an sich bekannter Weise — zum Beispiel mit Alkalialkoholaten, -amiden, -hydriden, -hydroxyden, -carbonaten oder quaternären Ammoniumverbindungen — gebildet werden. Als Säureakzeptor kommen anorganische oder tertiäre organische Basen, zum Beispiel Natriumhydroxyd, Kaliumhydroxyd, Kaliumcarbonat, Triäthylamin und Pyridin, in Betracht. Die Reaktion kann gegebenenfalls in Gegenwart eines Katalysators vorgenommen werden. Als Katalysator werden zum Beispiel Alkalihalogenide, vorzugsweise Alkalijodide, eingesetzt. Die Reaktionstemperatur kann innerhalb weiter Grenzen variieren, vorzugsweise arbeitet man zwischen 20 °C und dem Siedepunkt des verwendeten Lösungsmittels.

Gemäß der Verfahrensvariante f) werden die Äthinylbeziehungsweise Vinylverbindungen der allgemeinen Formel (X) durch katalytische Hydrierung reduziert. Als Hydrierkatalysator kommen Metalle, zum Beispiel Ruthenium, Palladium, Platin, Nickel, Eisen, Kupfer, Kobalt, Chrom, Zink, Molybdän oder Wolfram, in Frage, ferner die Oxyde und Sulfide der aufgeführten Metalle. Der Katalysator kann zum Beispiel hergestellt werden, indem man die Oxyde unmittelbar im Reaktionsgefäß mit Wasserstoff reduziert. Auf diese Weise geht man vor, wenn als Katalysator feinverteiltes Platin oder Palladium verwendet werden soll. Zur katalytischen Hydrierung können auch Katalysatoren verwendet werden, die vorher auf die Oberfläche eines Trägers aufgebracht wurden. Als Träger kommen zum Beispiel Tierkohle, Siliciumdioxyd, Aluminiumoxyd sowie die Sulfate und Carbonate der Erdalkalimetalle in Frage. Bevorzugt verwendet man als Katalysator Palladium, zweckmäßig in Form von Palladiumaktivkohle, oder Raneynickel. Die Reduktion erfolgt zweckmäßig in einem hinsichtlich der Reaktion inerten Lösungsmittel, zum Beispiel einem niederen aliphatischen Alkohol, in Äthern, Estern, aliphatischen, cycloaliphatischen und aromatischen Kohlenwasserstoffen oder in Gemischen der aufgeführten Lösungsmittel. Hydriert wird unter atmosphärischem oder erhöhtem Druck, vorzugsweise unterhalb 506 kPa, bei Temperaturen zwischen 20 °C und dem Siedepunkt des Reaktionsgemisches. Zweckmäßig hydriert man bei Raumtemperatur unter atmosphärischem Druck bis zur Beendigung der Wasserstoffaufnahme. Dann wird der Katalysator abfiltriert, das Filtrat eingedampft und das Produkt zum Beispiel durch Destillation oder Umkristallisation gereinigt.

Aus den Verbindungen der allgemeinen Formel (I) können gewünschtenfalls Säureadditionssalze oder quaternäre Salze gebildet werden. Zur Herstellung der Säureadditionssalze verwendet man anorganische oder organische Säuren, zum Beispiel Halogenwasserstoffsäuren, wie Salzsäure oder Bromwasserstoffsäure, Schwefelsäure, Phosphorsäuren, Ameisensäure, Essigsäure, Propionsäure, Oxalsäure, Glykolsäure, Maleinsäure, Fumarsäure, Bernsteinsäure, Weinsäure, Ascorbinsäure, Citronensäure, Apfelsäure, Salicylsäure, Milchsäure, Benzoesäure, Zimtsäure, Asparaginsäure, Glutaminsäure, N-Acetylglutaminsäure, Alkylsulfonsäuren, zum Beispiel Methansulfonsäure, Arylsulfonsäuren, zum Beispiel p-Toluolsulfonsäure. Zur Salzbildung wird zum Beispiel zu der mit einem inerten Lösungsmittel bereiteten Lösung der Verbindung der allgemeinen Formel (I), zum Beispiel einer äthanolischen Lösung, die entsprechende Säure gegeben, und das Salz wird mit einem mit Wasser nicht mischbaren Lösungsmittel, zum Beispiel mit Diäthyläther, ausgefällt. Zur Bildung der quaternären Salze werden vorzugsweise niedere Alkyl-, Alkenyl- oder aber Benzylhalogenide beziehungsweise Alkylsulfate verwendet. Die Umsetzung erfolgt in einem organischen Lösungsmittel, zweckmäßig in Aceton, Acetonitril, Äthanol oder deren Gemischen, bei Temperaturen zwischen Raumtemperatur und dem Siedepunkt des verwendeten Lösungsmittels. Das gebildete quaternäre Salz kann zum Beispiel durch Filtrieren abgetrennt und notwendigenfalls durch Umkristallisieren gereinigt werden.

Die erfindungsgemäßen Verbindungen der allgemeinen Formel (I) weisen wertvolle pharmakologische Eigenschaften auf. Sie verkürzen die Dauer der Äthylalkoholnarkose beziehungsweise haben eine antidepressive Wirkung. Die Verbindungen können deshalb zur Behandlung der akuten Alkoholintoxikation beziehungsweise zur Behandlung von Depressionszuständen eingesetzt werden.

Da Alkohol keine Substanz ist, welche durch Induzierung der Wirkung eines Enzymes schneller inaktiviert werden kann, hat die pharmakologische Wirksamkeit der Verbindungen der britischen Patentschrift 1 478 185 mit den pharmakologischen Wirkungen gegen akute Alkoholintoxikation und Depressionszustände der erfindungsgemäßen Verbindungen nichts zu tun. Auch haben die Verbindungen der genannten Druckschrift überhaupt keine Wirkung gegen akute Alkoholintoxikation und antidepressive Wirkung.

Die auf die durch Äthanol hervorgerufene Narkose ausgeübte Wirkung der Verbindungen wurde an

160-180 g schweren Hann.-Wistar-Ratten beiderlei Geschlechts untersucht. Jede Gruppe bestand aus 10 Tieren. Vor dem Versuch ließ man die Tiere 16 Stunden lang hungern. Die Behandlung erfolgte oral mit unterschiedlichen Dosen der Testverbindungen. Eine Stunde nach der Behandlung wurde den Tieren 3,5 g/kg Äthanol i. p. appliziert. Die Schlafdauer wurde vom Ausfall des Aufrichtreflexes (righting Reflex) bis zur spontanen Korrektur der Körperhaltung gemessen, dann der Durchschnitt für jede Gruppe errechnet und in Prozent der Kontrolle ausgedrückt. Als Referenzsubstanz wurde L-Cystein verwendet, das die durch Alkohol ausgelöste Narkose verkürzt. Die Kontrolle wurde mit Placebo und 3,5 mg/kg Äthanol behandelt. Die Ergebnisse sind in Tabelle 1 angegeben.

Verwendete Abkürzungen :
$\overline{X} \pm$ S.E. = Durchschnitt ± Standardabweichung
A = 3-Trifluormethyl-4'-[3-(diäthylamino)-propoxy]-α-äthyl-benzhydrol
B = 4-Chlor-4'-[2-(diäthylamino)-äthoxy]-α-äthyl-benzhydrol

Tabelle 1

| Behandlung | Dosis mg/kg | Schlafdauer (Kontrolle ± S.E., %) |
|---|---|---|
| A | 10,0 | 52 ± 11,1 |
| | 40,0 | 40 ± 5,0 |
| B | 10,0 | 48 ± 9,5 |
| | 40,0 | 39 ± 4,5 |
| L-Cystein | 500,0 | 63 ± 4,2 |
| Kontrolle | | 100 ± 6,5 |

Schlafdauer der Kontrolle : 83,0 ± 5,44 ($\overline{X} \pm$ S.E.) min

Die von den Verbindungen auf die durch Alkohol ausgelöste lokomotorische Hyperaktivität ausgeübte Wirkung wurde an 16-18 g schweren BALB/c-Mäusen beiderlei Geschlechts untersucht. Jede Gruppe bestand aus 15 Tieren. Die Testsubstanzen wurden oral verabreicht, eine Stunde vor der Verabrichung von Placebo (oral) beziehungsweise 2 g/kg Äthanol (intraperetoneal). Die Kontrolle erhielt Placebo. Die lokomotorische Aktivität der Tiere wurde mit dem Motimeter Animex BSE 2 Stunden lang gemessen. Die Ergebnisse, ausgedrückt in Prozent der Kontrolle, sind in der Tabelle 2 zusammengestellt.

Tabelle 2

| Behandlung | Dosis (mg/kg) Verbindung | $C_2H_5OH$ | lokomotorische Aktivität in 2 Stunden |
|---|---|---|---|
| Kontrolle (Placebo) | - | - | 100 ± 8,1 |
| Äthanol + Placebo | | 2000 | 190 ± 12,2 |
| B + Placebo | 10,0 | - | 110 ± 15,2 |
| B + Äthanol | 10,0 | 2000 | 110 ± 8,1 |

Die von den erfindungsgemäßen Verbindungen auf die durch Alkohol verursachte Ataxie und auf die Änderung des Muskeltonus ausgeübte Wirkung wurde am Drehstab untersucht. Trainierte und ausgewählte BALB/c-Mäuse beiderlei Geschlechts (Gewicht 16-18 g) wurden mit 40 mg/kg der Testverbindungen oral behandelt, eine Stunde vor der intraperetonealen Applikation von 2,5 g/kg Äthanol. Die Zeit, die die Tiere auf dem Drehstab zu bleiben vermochten, wurde 60, 90 und 120 Minuten nach der Verabreichung des Alkohols gemessen. Die Tiere, deren Koordination intakt war, blieben 120 Sekunden auf dem Drehstab. Jede Behandlung wurde an einer aus 10 Tieren bestehenden Gruppe vorgenommen. Die Ergebnisse sind in der folgenden Tabelle 3 zusammengestellt.

Tabelle 3

| Behandlung | Dosis mg/kg B | Dosis mg/kg Äthanol | auf der Drehstange gebliebene Tiere (%) nach 60 | 90 | 120 min |
|---|---|---|---|---|---|
| Äthanol | - | 2500 | 10 | 40 | 50 |
| B + Äthanol | 40,0 | 2500 | 40 | 70 | 90 |
| B | 40,0 | - | 100 | 100 | 100 |

Die antidepressive Wirkung der Verbindungen wurde durch Untersuchung der auf die durch Tetrabenazin induzierte Katalepsie ausgeübten Hemmwirkung und Messung der die durch Reserpin ausgelöste Hypothermie umkehrenden Wirkung bestimmt. Zur Bestimmung der anticholinergen Wirkung wurde die Hemmung des mittels Oxotremorin ausgelösten Tremors herangezogen. Als Vergleichssubstanz diente das in der Heilkunde allgemein angewendete Imipramin.

Zur Untersuchung der die Tetrabenazin-Katalepsie hemmenden Wirkung wurde Hann.-Wistar-Ratten von 150-160 g Gewicht oral die Testverbindung und eine Stunde später in einer Dosis von 30 mg/kg Tetrabenazin intraperitoneal appliziert. Die Hemmung der Katalepsie wurde 3 Stunden nach Verabreichung der Testverbindung kontrolliert. In der Tabelle 4 ist die aus dem Prozentsatz der nicht kataleptischen Tiere mittels Probit-Analyse berechnete $ED_{50}$-Dosis (zu 50 % wirksame Dosis) angegeben.

Die auf die Reserpin-Hypothermie ausgeübte umkehrende Wirkung wurde an männlichen CFLP-Mäusen des Gewichtes 18-22 g untersucht. Die Tiere wurden mit 5 mg/kg Reserpin i.p. behandelt. 16 Stunden nach der Verabreichung des Reserpins erhielten die Tiere die Testsubstanzen in unterschiedlichen Dosen oral verabreicht. Die rektale Temperatur der Tiere wurde vor der Behandlung mit Reserpin, 16 Stunden nach der Behandlung und 2 Stunden nach Verabreichung der Testverbindungen gemessen. In der Tabelle 4 ist diejenige Dosis angegeben, die bei den Tieren eine Erhöhung der Körpertemperatur um 50 % hervorrufen würde ($ED_{50}$).

Zur Bestimmung der anticholinergen Wirkung wurden CFLP-Mäusen männlichen Geschlechts (Gewicht : 18-22 g) i.p. mit 0,5 mg/kg Oxotremorin behandelt. 60 Minuten später wurden die Testverbindungen oral appliziert. In der Tabelle 4 sind die $ED_{50}$-Werte angegeben.

Die akute Toxizität der erfindungsgemäßen Verbindungen wurde an 160-180 g schweren Hann.-Wistar-Ratten beiderlei Geschlechts untersucht. Die Tiere erhielten eine einmalige Dosis von 500 mg/kg oral und wurden 14 Tage lang beobachtet. In der Tabelle 4 ist die Anzahl der verendeten Tiere angegeben.

C = 5-Trifluormethyl-4'-[2-(diäthylamino)-äthoxy]-α-äthylbenzhydrol

D = 3-Chlor-4'-[3-(diäthylamino)-propoxy]-α-äthyl-benzhydrol.

Tabelle 4

| | $ED_{50}$-Werte in mg/kg Katalepsiehemmung | Antireserpinwirkung | Oxotremorin-hemmung | Toxizität (Mortalität, %) |
|---|---|---|---|---|
| C | 20,0 | 30,0 | 160,0 wirkungslos | 0 |
| D | 18,8 | 17,0 | 160,0 wirkungslos | 0 |
| Imipramin | 13,7 | 15,0 | 50,0 | 80 |

Aus den Ergebnissen ist ersichtlich, daß die erfindungsgemäßen Verbindungen sowohl die deprimierende wie auch die stimulierende Wirkung des Alkohols auf das Zentralnervensystem bedeutend kompensieren. Die durch Alkohol hervorgerufene Narkose wird bedeutend verkürzt, wobei die Wirkung der Verbindungen in einer um 50mal geringeren Dosis die Wirkung der Referenzsubstanz L-Cystein erreicht beziehungsweise übertrifft. Die durch Alkohol ausgelöste Hyperaktivität wird von den Verbindungen normalisiert ; die lokomotorische Aktivität an sich wird nicht beeinflußt, die Ataxiejedoch bedeutend vermindert. Die antidepressive Wirkung der Verbindungen ist größenordnungsmäßig gleich der des Imipramins, sie ist jedoch nicht von den für die tricyclischen Antidepressiva, so auch für Imipramin charakteristischen Nebenwirkungen, insbesondere anticholinergen Nebenwirkungen, begleitet. Die Toxizität der Verbindungen ist geringer als die des Imipramins.

Die erfindungsgemäßen Verbindungen können zu Arzneimittelpräparaten zubereitet werden. Die

Präparate können oral, rektal und/oder parenteral verabreicht werden. Zur oralen Darreichung können Tabletten, Dragees oder Kapseln hergestellt werden. Bei der Herstellung von oral verabreichbaren Formen werden als Streckmittel zum Beispiel Milchzucker oder Stärke verwendet. Als Binde- und Granuliermittel kommen zum Beispiel Gelatine, Carboxymethylcellulose-natrium, Methylcellulose, Polyvinylpyrrolidon oder Stärkekleister in Frage. Als Sprengmittel werden in erster Linie Kartoffestärke oder mikrokristalline Cellulose zugesetzt, jedoch können beispielsweise auch Ultraamylopektin oder Formaldehydcasein verwendet werden. Als Antihaftmittel und Gleitmittel kommen z. B. Talk, kolloidale Kieselsäure, Stearin, Ca- und Mgstearat in Frage.

Die Tabletten können zum Beispiel durch Naßgranulieren und anschließendes Pressen hergestellt werden. Das Gemisch aus Wirkstoff und Füllstoffen sowie gegebenenfalls ein Teil des Sprengmittels werden mit der wäßrigen, alkoholischen oder wäßrig-alkoholischen Lösung der Bindemittel in einer geeigneten Vorrichtung granuliert, und das Granulat wird getrocknet. Zu dem trockenen Granulat werden der Rest des Sprengmittels, ferner die Antihaftmittel und das Gleitmittel gegeben, und das Gemisch wird zu Tabletten gepreßt. Gegebenenfalls können die Tabletten zur Erleichterung der Dosierung mit Teilungsmarkierungen versehen werden. Die Tabletten können auch unmittelbar durch Pressen eines Gemisches aus dem Wirkstoff und geeigneten Hilfsstoffen hergestellt werden.

Die Tabletten können gegebenenfalls unter Verwendung der in der Arzneimittelindustrie üblichen Schutz-Geschmacks- und Farbstoffe, zum Beispiel Zucker, Cellulosederivate, wie Methyl- oder Äthylcellulose oder Carboxymethylcellulose-natrium, Polyvinylpyrrolidon, Calciumphosphat, Calciumcarbonat, Lebensmittelfarbstoffe, Lebensmittelfarblacke, Aromastoffe und Eisenoxydpigmente, auf die übliche Weise dragiert werden.

Zur Herstellung von Kapseln wird das Gemisch aus Wirkstoff und Füllstoffen in Kapseln gefüllt.

Zur rektalen Anwendung werden Suppositorien hergestellt. Diese enthalten außer dem Wirkstoff eine Trägermasse, das sog. Adeps pro suppositori. Als dieses kommen Pflanzenfette, zum Beispiel gehärtete Pflanzenöle, die Triglyceride von Fettsäuren mit 12-18 Kohlenstoffatomen, vorzugsweise die Trägerstoffe der Markenbezeichnung Witepsol®, in Betracht. Der Wirkstoff wird in der geschmolzenen Trägermasse homogen verteilt und das erhaltene Gemisch zu Suppositorien vergossen.

Zur parenteralen Verabreichung werden Präparate in Injektionslösungsform hergestellt. Dazu werden die Wirkstoffe, gegebenenfalls in Gegenwart von Lösungsvermittlern, wie Polyoxyäthylensorbitmonolaurat, -monooleat oder -monostearat (Tween 20®, Tween 60®, Tween 80®), in destilliertem Wasser und/oder unterschiedlichen organischen Lösungsmitteln, zum Beispiel Glykoläthern, gelöst. Die Injektionslösungen können ferner verschiedene Hilfsstoffe, zum Beispiel Konservierungsstoffe, Benzylalkohol, p-Oxybenzcesäuremethyl- oder -propylester, Benzalkoniumchlorid oder Phenylmercuriborat, zum Binden von Metallspuren Komplexbildner, z. B. Äthylendiamin-tetraessigsäure, Puffersubstanzen oder Stoffe zum Einstellen des pH-Wertes sowie gegebenenfalls lokalanästhetisch wirksame Substanzen, wie Lidocain, enthalten. Die Injektionslösungen werden vor dem Abfüllen filtriert und, nachdem sie in Ampullen gefüllt wurden, sterilisiert.

Die Tagesdosis beträgt, abhängend vom Zustand des Kranken, 0,1-300 mg/kg, vorzugsweise 2,0-160 mg/kg, und wird zweckmäßig in kleineren Einzeldosen verabreicht. Für antidepressiv wirkende Verbindungen beträgt die Tagesdosis, abhängend vom Zustand des Kranken, 0,5-100 mg/kg, vorzugsweise 1,5-20 mg/kg, und wird in Einzeldosen verabreicht.

Die Erfindung wird an Hand der folgenden Beispiele näher erläutert.

## Beispiel 1

3-Trifluormethyl-4'-[3-(diäthylamino)-propoxy]-α-äthylbenzhydrol.

Zu dem aus 3,7 g Magnesiumspänen und 33,8 g 3-(Trifluormethyl)-brombenzol in 100 ml Tetrahydrofuran bereiteten Grignard-Reagens wird unter ständigem Rühren und schwachem Sieden am Rückfluß tropfenweise die Lösung von 26,3 g 4-[3-(Diäthylamino)-porpoxy]-propiophenon in 60 ml Tetrahydrofuran gegeben. Das Reaktionsgemisch wird weitere 30 Minuten lang eben am Sieden erhalten, dann abgekühlt und in gesättigte wäßrige Ammoniumchloridlösung gegossen. Die wäßrige Phase wird mit Tetrahydrofuran extrahiert. Die vereinigten organischen Phasen werden mit gesättigter wäßriger Kochsalzlösung gewaschen, über wasserfreiem Magnesiumsulfat getrocknet und schließlich unter vermindertem Druck eingedampft. Der Rückstand wird im Vakuum destilliert. 24,4 g-der Titelverbindung werden erhalten. Siedepunkt : 186-188 °C/13,3 Pa, Schmelzpunkt : 44-46 °C.

Elementaranalyse für die Summenformel $C_{23}H_{30}F_3NO_2$

berechnet : C 67,46 H 7,38 F 13,92 N 3,42 %

gefunden : C 67,57 H 7,33 F 14,10 N 3,56 %.

Zu der ätherischen Lösung der Base wird unter Kühlen salzsaurer Äther gegeben. Das ausgefallene Hydrochlorid wird abfiltriert, mit Äther gewaschen und dann getrocknet. Schmelzpunkt : 141-143 °C.

Die äthanolische Lösung der Base wird mit der äthanolischen Lösung von Citronensäure behandelt. Nach Zusatz von Äther fällt das Dihydrocitrat aus. Es wird abfiltriert, mit Äther gewaschen und dann getrocknet. Schmelzpunkt : 62 °C.

Durch entsprechende Wahl der Ausgangsverbindungen werden folgende Produkte hergestellt.

2-Methoxy-4'-[2-(diäthylamino)-äthoxy]-α-äthylbenzhydrol, Schmelzpunkt : 51-52 °C.

Elementaranalyse für die Summenformel $C_{22}H_{31}NO_3$
berechnet: C 73,91 H 8,74 N 3,92 %
gefunden: C 73,83 H 8,83 N 4,11 %.

4-Trifluormethyl-4'-[2-(diäthylamino)-äthoxyl]-α-äthylbenzhydrol, Schmelzpunkt: 56-57 °C
Elementaranalyse für die Summenformel $C_{22}H_{28}F_3NO_2$
berechnet: C 66,81 H 7,14 F 14,41 N 3,54 %
gefunden: C 66,83 H 7,22 F 14,60 N 3,67 %
Schmelzpunkt des Hydrochlorids: 152-153 °C
Schmelzpunkt des Dihydrocitrats: 110-111 °C.

## Beispiel 2

3-Chlor-4'-[3-(diäthylamino)-propoxy]-α-äthylbenzhydrol.

Ein Gemisch aus 19,2 g 3-Chlor-4'-(3-brompropoxy)-α-äthylbenzhydrol und 51 ml Diäthylamin wird am Rückfluß unter Rühren 8 Stunden lang gekocht und das Reaktionsgemisch dann im Vakuum eingedampft. Der Rückstand wird mit Wasser versetzt und mit Benzol extrahiert. Die organische Phase wird mit Wasser gewaschen und nach dem Trocknen über wasserfreiem Magnesiumsulfat unter vermindertem Druck eingedampft. Man erhält 11,5 g der Titelverbindung. Siedepunkt: 203-206 °C/13,3 Pa.

Elementaranalyse für die Summenformel $C_{22}H_{30}ClNO_2$
berechnet: C 70,28 H 8,04 Cl 9,43 N 3,73 %
gefunden: C 70,35 H 8,15 Cl 9,35 N 3,84 %.

## Beispiel 3

3-Chlor-4'-[2-(diäthylamino)-äthoxy]-α-äthylbenzhydrol.

Zu 125 ml einer 0,8-molaren ätherischen Äthyllithiumlösung wird unter Argonatmosphäre bei einer Temperatur zwischen -20 und -15 °C unter ständigem Rühren tropfenweise die Lösung von 13,3 g 3-Chlor-4'-[2-(diäthylamino)-äthoxy]-benzophenon in 160 ml Äther gegeben. Das Reaktionsgemisch wird bei Raumtemperatur eine Stunde lang gerührt und dann unter Kühlung in gesättigte wäßrige Ammoniumchloridlösung eingegossen. Die wäßrige Phase wird mit Äther extrahiert. Die ätherischen Phasen werden vereinigt, mit Wasser gewaschen, über wasserfreiem Magnesiumsulfat getrocknet und unter vermindertem Druck vom Lösungsmittel befreit. Der feste Rückstand wird aus n-Hexan kristallisiert. Man erhält 8,9 g der Titelverbindung, die bei 64-65 °C schmilzt.

Elementaranalyse für die Summenformel $C_{21}H_{28}ClNO_2$
berechnet: C 69,69 H 7,80 Cl 9,80 N 3,87 %
gefunden: C 69,77 H 7,85 Cl 9,94 N 3,98 %.

## Beispiel 4

4-Fluor-4'-[3-(diäthylamino)-propoxy]-α-äthylbenzhydrol

Zu dem aus 2,4 g Magnesiumspänen und 28,6 g 4-[3-(Diäthylamino)-propoxy]-brombenzol in 100 ml wasserfreiem Tetrahydrofuran bereiteten Grignard-Reagens wird bei 20 °C tropfenweise die Lösung von 12,2 g 4-Fluorpropiophenon in 60 ml Tetrahydrofuran gegeben. Das Reaktionsgemisch wird eine Stunde lang schwach gekocht und nach dem Abkühlen in 20 %ige wäßrige Ammoniumchloridlösung eingegossen. Das Tetrahydrofuran wird unter vermindertem Druck abdestilliert und der Rückstand mit Benzol extrahiert. Die organische Phase wird mit Wasser neutral gewaschen, über wasserfreiem Magnesiumsulfat getrocknet und nach dem Filtrieren unter vermindertem Druck eingedampft. Der Rückstand wird im Vakuum destilliert. Man erhält 19,5 g der Titelverbindung. Siedepunkt: 193-195 °C/13,3 Pa.

Elementarnalyse für die Summenformel $C_{22}H_{30}FNO_2$
berechnet: C 73,50 H 8,41 F 5,28 N 3,90 %
gefunden: C 73,66 H 8,51 F 5,43 N 3,81 %.
Das Dihydrocitrat schmilzt bei 64-66 °C.

## Beispiel 5

2-Methoxy-4'-[3-(diäthylamino)-propoxy]-α-äthylbenzhydrol.

12,9 g 2-Methoxy-4'-hydroxy-α-äthylbenzhydrol werden in 100 ml Methylisobutylketon gelöst. Die Lösung wird nach Zusatz von 23 g wasserfreiem Kaliumcarbonat, 0,4 ml 40 %iger Tetrabutylammoniumhydroxydlösung und 10,3 g 3-Diäthylaminopropylchlorid-hydrochlorid 4 Stunden lang gekocht. Dann wird das Lösungsmittel unter vermindertem Druck abdestilliert. Der Rückstand wird mit Wasser versetzt und mit Benzol extrahiert. Die organische Phase wird zuerst mit 5 %iger Kaliumhydroxydlösung, dann mit Wasser gewaschen, über wasserfreiem Kaliumcarbonat getrocknet und unter vermindertem Druck vom Lösungsmittel befreit. Der Rückstand wird im Vakuum fraktioniert. Man erhält 13,2 g der Titelverbindung. Siedepunkt: 200-203 °C/13,3 Pa.

Elementaranalyse für die Summenformel $C_{23}H_{33}NO_3$
berechnet : C 74,35 H 8,99 N 3,77 %
gefunden : C 74,48 H 9,14 N 3,69 %.
Schmelzpunkt des Dihydrocitrats : 79 °C.

Beispiel 6

2,5-Dimethyl-4'-[2-(diäthylamino)-äthoxy]-α-äthylbenzhydrol.

10,5 g 2,5-Dimethyl-4'-[2-(diäthylamino)-äthoxy]-α-äthinyl-benzhydrol werden in 110 ml Methanol gelöst und nach Zusatz von 0,5 g 10 %iger Palladiumaktivkohle bis zur Aufnahme der berechneten Wasserstoffmenge hydriert. Der Katalysator wird abfiltriert und das Filtrat unter vermindertem Druck eingedampft. Der feste Rückstand wird aus einem Gemisch von n-Hexan und Äthylacetat umkristallisiert. Man erhält 9,1 g der Titelverbindung, die bei 89-90 °C schmilzt.

Elementaranalyse für die Summenformel $C_{23}H_{33}NO_2$
berechnet : 77,:70 H 9,35 N 3,94 %
gefunden : C 77,64 H 9,51 N 4,11 %.

Beispiel 7

4-Chlor-4'-[3-(diäthylamino]-propoxy]-α-äthylbenzhydrol.

Zu dem aus 7,2 g Magnesiumspänen und 32,6 g Äthylbromid in 120 ml wasserfreiem Äther hergestellten Grignard-Reagens wird bei -30 °C tropfenweise die Lösung von 25,6 g 4-Chlor-4'-[3-(diäthylamino)-propoxy]-benzophenon in 300 ml wasserfreiem Äther gegeben. Das Reaktionsgemisch wird am Rückfluß eine halbe Stunde lang gekocht und nach dem Abkühlen in eiskalte wäßrige Ammoniumchloridlösung gegossen. Die wäßrige Phase wird mit Äther extrahiert. Die ätherische Phase wird mit Wasser neutral gewaschen, über wasserfreiem Magnesiumsulfat getrocknet und unter vermindertem Druck eingedampft. Der Rückstand wird im Vakuum fraktioniert. Man erhält 15,3 g der Titelverbindung.

Siedepunkt : 212-214 °C/13,3 Pa.
Elementaranalyse für die Summenformel $C_{22}H_{30}ClNO_2$
berechnet : C 70,28 H 8,04 Cl 9,43 N 3,73 %
gefunden : C 70,33 H 8,11 Cl 9,51 N 3,85 %.
Das Dihydrocitrat schmilzt bei 62-63 °C.

Beispiel 8

3-Trifluormethyl-4'-[2-(diäthylamino)-äthoxyl]-α-äthylbenzhydrol.

Zu 250 ml einer 0,4 molaren ätherischen 3-Trifluormethylphenyllithiumlösung wird unter Argonatmosphäre bei -30 °C tropfenweise die Lösung von 6,3 g 4-[2-(Diäthylamino)-äthoxy]-propiophenon in 60 ml Äther zugegeben und das Reaktionsgemisch bei Raumtemperatur 2 Stunden lang gerührt. Nach dem Abkühlen wird das Reaktionsgemisch in gesättigte wäßrige Ammoniumchloridlösung gegossen und die wäßrige Phase mit Äther extrahiert. Die vereinigten ätherischen Phasen werden mit Wasser neutral gewaschen, über wasserfreiem Magnesiumsulfat getrocknet und unter vermindertem Druck vom Lösungsmittel befreit. Der Rückstand wird mit einem im Volumverhältnis 7 : 3 bereiteten Gemisch von Benzol und Äthylacetat an einer Silikagelsäule chromatographiert. Das Lösungsmittelgemisch wird im Vakuum abdestilliert und der Rückstand aus n-Hexan kristallisiert. Nan erhält 3,2 g der Titelverbindung, die bei 74-75 °C schmilzt.

Elementaranalyse für die Summenformel $C_{22}H_{28}F_3NO_2$
berechnet : C 66,81 H 7,14 F 14,41 N 3,54 %
gefunden : C 66,93 H 7,03 F 14,58 N 3,41 %.

Beispiel 9

4-Chlor-4'-[2-(diäthylamino)-äthoxy]-α-äthylbenzhydrol.

17,9 g 4-Chlor-4'-[2-(diäthylamino)-äthoxy]-α-äthinyl-benzhydrol werden in 180 ml Benzol gelöst und nach Zugabe von 0,9 g 10 %iger Palladiumaktivkohle bis zur Aufnahme der berechneten Menge wasserstoff hydriert. Nach Abfiltrieren des Katalysators wird das Benzol unter vermindertem Druck abdestilliert. Der feste Rückstand wird aus n-Hexan kristallisiert. Man erhält 14,2 g der Titelverbindung, die bei 50-51 °C schmilzt.

Elementaranalyse für die Summenformel $C_{21}H_{28}ClNO_2$
berechnet : C 69,69 H 7,80 Cl 9,80 N 3,87 %
gefunden : C 69,75 H 7,78 Cl 9,91 N 3,77 %.
Das Hydrochlorid schmilzt bei 153-154 °C,
das Dihydrocitrat bei 97-98 °C und
das Hydrofumarat bei 71-73 °C.

Beispiel 10

4-Chlor-4′-[2-(diäthylamino)-äthoxy]-α-äthylbenzhydrol-äthojodid.

Die Lösung von 3,6 g 4-Chlor-4′-[2-(diäthylamino)-äthoxy]-α-äthylbenzhydrol und 1,2 ml Äthyljodid in 18 ml wasserfreiem Aceton wird am Rückfluß 2 Stunden lang gekocht. Nach dem Abkühlen wird das Reaktionsgemisch mit Äther verdünnt. Das kristallin ausfallende Produkt wird abfiltriert und getrocknet. Man erhält 4,3 g der Titelverbindung, die bei 143-144 °C schmilzt.

Analog wird das 4-Trifluormethyl-4′-[2-(diäthylamino)-äthoxy]-α-äthylbenzhydrol-äthojodid hergestellt, das bei 167-168 °C schmilzt.

Beispiel 11

Aus den erfindungsgemäßen Verbindungen können zum Beispiel die folgenden Arzneimittelpräparate hergestellt werden.

Tabletten

Zusammensetzung einer Tablette :

| | |
|---|---|
| Wirkstoff | 100,0 mg |
| Lactose | 184,0 mg |
| Kartoffelstärke | 80,0 mg |
| Polyvinylpyrrolidon | 8,0 mg |
| Talk | 12,0 mg |
| Magnesiumstearat | 2,0 mg |
| Aerosil® (kolloidales $SiO_2$) | 2,0 mg |
| Ultraamylopektin | 12,0 mg |

Durch Naßgranulieren und Pressen werden aus den angegebenen Stoffen Tabletten von 400 mg Gewicht hergestellt.

Wirkstoff : 4-Trifluormethyl-4′-[2-(diäthylamino)-äthoxy]-α-äthyl-benzhydrol.

Dragees

Die auf die beschriebene Weise erhaltenen Tabletten werden in an sich bekannter Weise mit einem Zucker und Talk bestehenden Überzug versehen und dann mit einem Gemisch aus Bienenwachs und Carnaubawachs poliert. Gewicht eines Dragees 500,0 mg

Kapseln

Zusammensetzung der Füllung einer Kapsel

| | |
|---|---|
| Wirkstoff | 50,0 mg |
| Lactose | 100,0 mg |
| Talk | 2,0 mg |
| Kartoffelstärke | 30,0 mg |
| mikrokristalline Cellulose | 3,0 mg |

Der Wirkstoff wird gründlich mit den Hilfsstoffen vermischt, das Gemisch durch ein Sieb der Maschenweite 0,32 mm gesiebt und dann in Hartgelatinekapseln (Größe 4) gefüllt.

Wirkstoff : 4-Chlor-4′-[2-(diäthylamino)-äthoxy]-α-äthylbenzhydrol

Suppositorien

Zusammensetzung eines Suppositoriums :

| | |
|---|---|
| Wirkstoff | 100,0 mg |
| Lactose | 200,0 mg |
| Suppositorienmasse (z. B. Witepsol®H) | 1 700 mg |

Die Grundmasse wird geschmolzen und die Schmelze auf 35 °C gekühlt. Der Wirkstoff wird gründlich mit der Lactose vermischt und das Gemisch in einem Homogenisator mit der Schmelze homogenisiert. Das erhaltene Gemisch wird in gekühlte Suppositorienformen gegossen. Gewicht eines Suppositoriums : 2 000 mg.

Wirkstoff : 3-Trifluormethyl-4′-[3-(diäthylamino)-propoxyl-α-äthylbenzhydrol.

Suspension

100 ml Suspension enthalten :

| | |
|---|---|
| Wirkstoff | 1,0 g |

| | |
|---|---|
| Natriumhydroxyd | 0,26 g |
| Citronensäure | 0,30 g |
| Nipagin® (Natriumsalz des 4-Hydroxybenzoesäuremethylesters) | 0,10 g |
| Carbopol® 940 (Polyacrylsäure) | 0,30 g |
| Äthanol (96 %ig) | 1,00 g |
| Himbeeraroma | 0,60 g |
| Sorbit (70 %ige wäßrige Lösung) | 71,00 g |
| dest. Wasser zum Auffüllen auf | 100,00 ml |

Das Nipagin® und die Citronensäure werden in 20 ml destilliertem Wasser gelöst. Zu der Lösung gibt man in kleinen Portionen, unter intensivem Rühren das Carbopol® und läßt die Lösung dann 10-12 Stunden lang stehen. Anschließend werden die mit 1 ml destilliertem Wasser bereitete Lösung des Natriumhydroxyds, dann die wäßrige Lösung des Sorbits und schließlich die äthanolische Lösung des Himbeeraromas unter Rühren zugegeben. Zu der so bereiteten Trägersubstanz gibt man in kleinen Portionen den Wirkstoff und homogenisiert das Ganze mit einem Mixer. Die Suspension wird mit destilliertem Wasser auf 100 ml aufgefüllt und in einer Kolloidmühle homogenisiert.

Wirkstoff : 3-Chlor-4'-[3-(diäthylamino)-propoxy]-α-äthylbenzhydrol.

**Patentansprüche** (für die Vertragsstaaten : BE, CH, DE, FR, GB, IT, LI, LU, NL, SE)

1. [ω-(Diäthylamino)-alkoxy]-α-[äthyl]-benzhydrolderivate der allgemeinen Formel

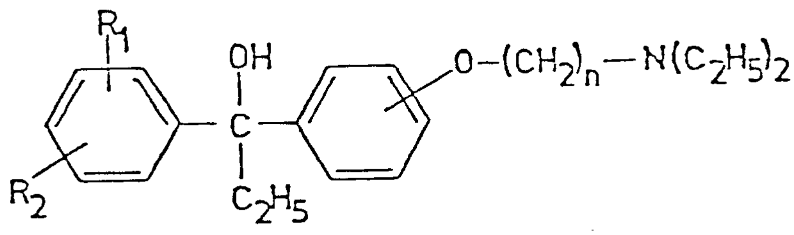

(I)

worin

$R_1$ für ein Wasserstoffatom, ein Halogenatom, einen Trihalogenmethylrest oder einen geradkettigen oder verzweigten Alkyl- oder Alkoxyrest mit je 1 bis 4 Kohlenstoffatom(en) steht,

$R_2$ ein Halogenatom, einen Trihalogenmethyl- rest oder einen Alkyl- oder Alkoxyrest mit je 1 bis 4 Kohlenstoffatom(en) bedeutet und

n eine ganze Zahl von 1 bis 4 ist,

sowie ihre Säureadditionssalze und quaternären Salze.

2. [ω-(Diäthylamino)-alkoxy]-α-[äthyl]-benzhydrolderivate nach Anspruch 1, dadurch gekennzeichnet, daß das, beziehungsweise die, Halogenatom(e), für das, beziehungsweise die, $R_1$ und/oder $R_2$ stehen kann, beziehungsweise können, [ein] Chlor-, Fluor- und/oder Bromatom(e) ist, beziehungsweise sind.

3. [ω-(Diäthylamino)-alkoxy]-α-[äthyl]-benzhydrolderivate nach Anspruch 1 oder 2, dadurch gekennzeichnet, daß der, beziehungsweise die, Trihalogenmethylrest(e), für den, beziehungsweise die, $R_1$ und/oder $R_2$ stehen kann, beziehungsweise können, [ein] Trifluormethylrest(e) und/oder Trichlormethylrest(e), ist, beziehungsweise sind.

4. [ω-(Diäthylamino)-alkoxy]-α-[äthyl]-benzhydrolderivate nach Anspruch 1 bis 3, dadurch gekennzeichnet, daß der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind.

5. [ω-(Diäthylamino)-alkoxy]-α-[äthyl]-benzhydrolderivate nach Anspruch 1 bis 4, dadurch gekennzeichnet, daß die Alkylreste, für die $R_3$ und $R_4$ stehen, solche mit 3 oder 4, insbesondere 3, Kohlenstoffatomen sind.

6. [ω-(Diäthylamino)-alkoxy]-α-[äthyl]-benzhydrolderivate nach Anspruch 1 bis 5, dadurch gekennzeichnet, daß n 2 bis 4, insbesondere 2 oder 3, ist.

7. Die [ω-(Diäthylamino)-alkoxy]-α-[äthyl]-benzhydrolderivate 3-Trifluormethyl-4'-[3-(diäthylamino)-propoxy]-α-äthyl-benzhydrol, 2-Methoxy-4'-[2-(diäthylamino)-äthoxy]-α-äthyl-benzhydrol, 4-Trifluormethyl-4'-[2-(diäthylamino)-äthoxy]-α-äthyl-benzhydrol, 3-Chlor-4'-[3-(diäthylamino)-propoxy]-α-äthyl-benzhydrol, 3-Chlor-4'-[2-(diäthylamino)-äthoxy]-α-äthyl-benzhydrol, 4-Fluor-4'-[3-(diäthylamino)-propoxy]-α-äthyl-benzhydrol, 2-Methoxy-4'-[3-(diäthylamino)-propoxyl]-α-äthyl-benzhydrol, 2,5-Dimethyl-4'-[2-(diäthylamino)-äthoxy]-α-äthyl-benzhydrol, 4-Chlor-4'-[3-(diäthylamino)-propoxy]-α-äthyl-benzhydrol, 3-Trifluormethyl-4'-[2-(diäthylamino)-äthoxy]-α-äthyl-benzhydrol und 4-Chlor-4'-[2-(diäthylamino)-äthoxy]-α-äthyl-benzhydrol sowie ihre Salze.

8. Verfahren zur Herstellung der Verbindungen nach Anspruch 1 bis 7, dadurch gekennzeichnet, daß man

a) am Benzolring ω-(diäthylamino)-alkoxysubstituierte Propiophenone der allgemeinen Formel

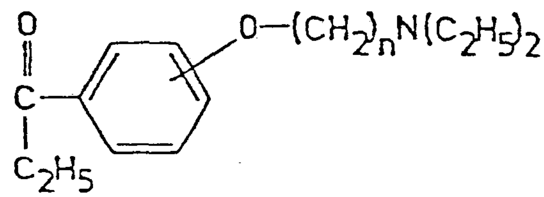

(II)

worin n wie im Anspruch 1 oder 6 festgelegt ist, mit eine Phenylgruppe aufweisenden metallorganischen Verbindungen der allgemeinen Formel

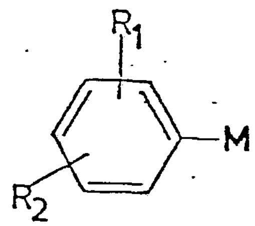

(III)

worin

$R_1$ und $R_2$ wie in den Ansprüchen 1 bis 4 festgelegt ist und

M für ein Alkalimetallatom, vorzugsweise Lithium-, Natrium- oder Kaliumatom oder einen Rest der Formel MgX mit X = Halogenatom steht,

umsetzt oder

b) [ω-(Halogen)-alkoxy]-α-[äthyl]-benzhydrolderivate der allgemeinen Formel

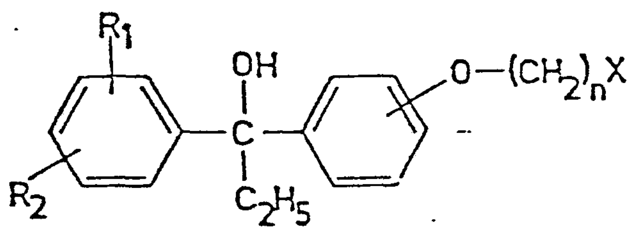

(IV)

worin

$R_1$, $R_2$ und n wie in den Ansprüchen 1 bis 4 oder 6 festgelegt sind und

X für ein Halogenatom steht,

mit Diäthylamin umsetzt oder

c) [ω-(Diäthylamino)-alkoxy]-benzophenonderivate der allgemeinen Formel

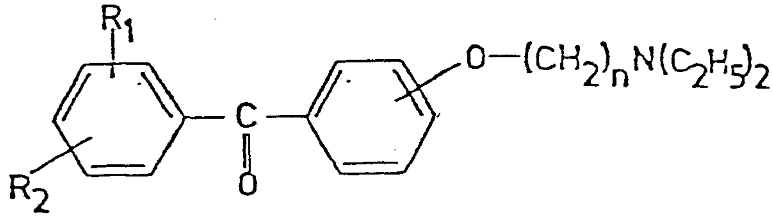

(V)

worin $R_1$, $R_2$ und n wie in den Ansprüchen 1 bis 4 oder 6 festgelegt sind, mit eine Äthylgruppe aufweisenden metallorganischen Verbindungen, insbesondere Äthylmagnesiumhalogeniden oder Äthyllithium, umsetzt oder

d) am Benzolring substituierte Propiophenone der allgemeinen Formel

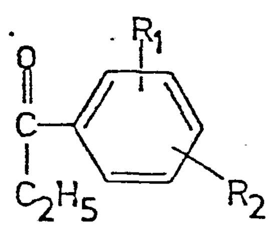

(VI)

worin $R_1$ und $R_2$ wie in den Ansprüchen 1 bis 4 festgelegt sind, inst eine {[ω-(Diäthylamino)-alkoxy]-phenyl}-gruppe aufweisenden Grignard-Verbindungen der allgemeinen Formel

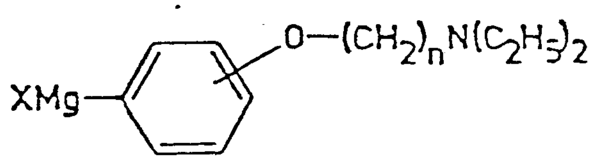

(VII)

worin die Bedeutung von

n wie im Anspruch 1 oder 6 festgelegt ist und

X für ein Halogenatom steht,

umsetzt oder

e) [Hydroxy]-α-[äthyl]-benzhydrolderivate der allgemeinen Formel

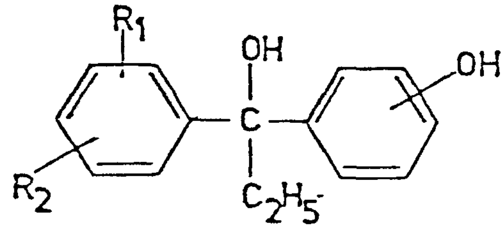

(VIII)

worin $R_1$ und $R_2$ wie in den Ansprüchen 1 bis 4 festgelegt sind, vorzugsweise in Form ihrer Alkaliphenolate oder quaternären Ammoniumphenolate, mit tertiären Aminen der allgemeinen Formel

$$(C_2H_5)_2N{-}(CH_2)_n{-}X \qquad (IX)$$

worin

X für ein Alkylsulfonyloxy- oder Arylsulfonyloxyrest oder ein Halogenatom steht und

n wie im Anspruch 1 oder 6 festgelegt ist, oder mit deren Salzen, vorzugsweise in Gegenwart von säurebindenden Mitteln,

umsetzt oder

f) [ω-(Diäthylamino)-alkoxy]-α-[äthinyl]-beziehungsweise [ω-(Diäthylamino)-alkoxy]-α-[vinyl]-benzhydrole der allgemeinen Formel

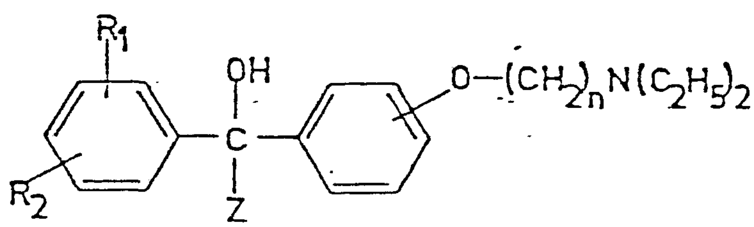

(X)

worin

$R_1$, $R_2$ und n wie in den Ansprüchen 1 bis 4 oder 6 festgelegt sind und

Z für einen Äthinyl- oder Vinylrest steht,

reduziert, worauf man gegebenenfalls in an sich bekannter Weise die erhaltenen [ω-(Diäthylamino)-alkoxy]-α-[äthyl]-benzhydrolderivate der allgemeinen Formel I mit Säuren in Säureadditionssalze oder mit Quaternisierungsmitteln in quaternäre Salze überführt oder gegebenenfalls die erhaltenen Säureadditionssalze der [ω-(Diäthylamino)-alkoxy]-α-[äthyl]-benzhydrolderivate der allgemeinen Formel I in die entsprechenden freien Basen der allgemeinen Formel I und/oder in andere Säureadditionssalze überführt.

9. Arzneimittel, gekennzeichnet durch einen Gehalt an 1 oder mehr Verbindung(en) nach Anspruch 1 bis 7 als Wirkstoff(e), zweckmäßigerweise zusammen mit 1 oder mehr üblichen pharmazeutischen Konfektionierungsmittel(n).

**Patentansprüche** (für den Vertragsstaat AT)

1. Verfahren zur Herstellung von [ω-(Diäthylamino)-alkoxy]-α-[äthyl]-benzhydrolderivaten der allgemeinen Formel

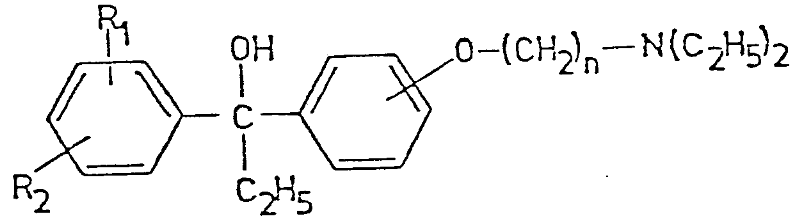

(I),

worin

$R_1$ für Wasserstoffatom, ein Halogenatom, einen Trihalogenmethylrest oder einen geradkettigen oder verzweigten Alkyl- oder Alkoxyrest mit je 1 bis 4 Kohlenstoffatom(en) steht,

$R_2$ ein Halogenatom, einen Trihalogenmethylrest oder einen Alkyl- oder Alkoxyrest mit je 1 bis 4 Kohlenstoffatom(en) bedeutet und

n eine ganze Zahl von 1 bis 4 ist,

sowie ihren Säureadditionssalzen und quarternären Salzen, dadurch gekennzeichnet, daß man

a) am Benzolring ω-(diäthylamino)-alkoxysubstituierte Propiophenone der allgemeinen Formel

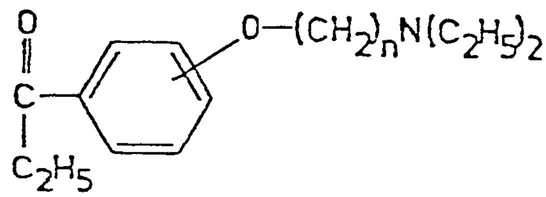

(II),

worin n wie oben festgelegt ist, mit eine Phenylgruppe aufweisenden metallorganischen Verbindungen der allgemeinen Formel

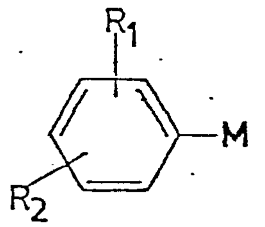

(III),

worin

$R_1$ und $R_2$ wie oben festgelegt sind und

M für ein Alkalimetallatom, vorzugsweise Lithium-, Natrium- oder Kaliumatom oder einen Rest der Formel MgX mit X = Halogenatom steht,

umsetzt oder

b) [ω-(Halogen)-alkoxy]-α-[äthyl]-benzhydrolderivate der allgemeinen Formel

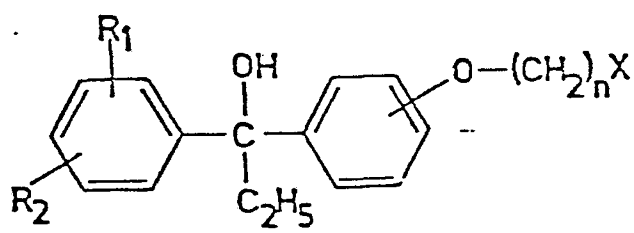

(IV),

worin

$R_1$, $R_2$ und n wie oben festgelegt sind und

X für ein Halogenatom steht,

mit Diäthylamin umsetzt oder

c) [ω-(Diäthylamino)-alkoxy]-benzophenonderivate der allgemeinen Formel

(Voir Dessin p. 17)

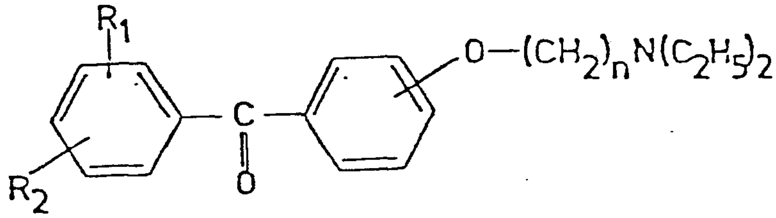

(V),

worin $R_1$, $R_2$ und n wie oben festgelegt sind, mit eine Äthylgruppe aufweisenden metallorganischen Verbindungen, insbesondere Äthylmagnesiumhalogeniden oder Äthyllithium, umsetzt oder

d) am Benzolring substituierte Propiophenone der allgemeinen Formel

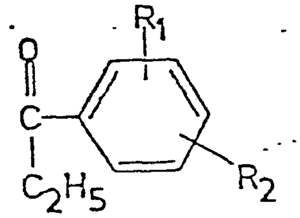

(VI),

worin $R_1$ und $R_2$ wie oben festgelegt sind, mit eine {[ω-(Diäthylamino)-alkoxy-phenyl}-gruppe aufweisenden Grignard-Verbindungen der allgemeinen Formel

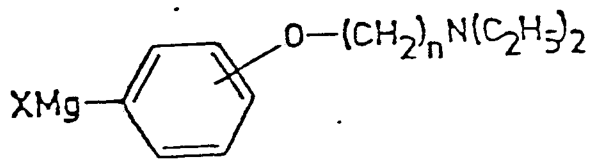

(VII)

worin die Bedeutung von

n wie oben festgelegt ist und

X für ein Halogenatom steht,

umsetzt oder

e) [Hydroxy]-α-[äthyl]-benzhydrolderivate der allgemeinen Formel

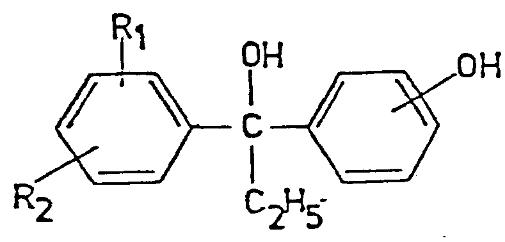

(VIII),

worin $R_1$ und $R_2$ wie oben festgelegt sind, vorzugsweise in Form ihrer Alkaliphenolate oder quaternären Ammoniumphenolate, mit tertiären Aminen der allgemeinen Formel

$$(C_2H_5)_2N—(CH_2)_n—X \quad \text{(IX)},$$

worin

X für ein Alkylsulfonyloxy- oder Arylsulfonyloxyrest oder ein Halogenatom steht und

n wie oben festgelegt ist, oder mit deren Salzen, vorzugsweise in Gegenwart von säurebindenden Mitteln,

umsetzt oder

f) [ω-(Diäthylamino)-alkoxy]-α-[äthinyl]-beziehungsweise [ω-(Diäthylamino)-alkoxy-α-[vinyl]-benzhydrole der allgemeinen Formel

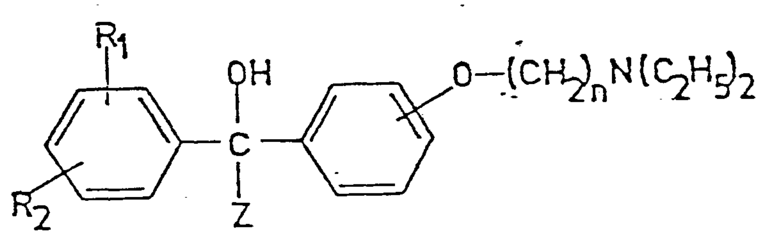

(X),

worin

$R_1$, $R_2$ und n wie oben festgelegt sind und

Z für einen Äthinyl- oder Vinylrest steht,

reduziert, worauf man gegebenenfalls in an sich bekannter Weise die erhaltenen [ω-(Diäthylamino)-alkoxy]-α-[äthyl]-benzhydrolderivate der allgemeinen Formel I mit Säuren in Säureadditionssalze oder mit Quaternisierungsmitteln in quaternäre Salze überführt oder gegebenenfalls die erhaltenen Säureadditionssalze der [ω-(Diäthylamino)-alkoxy]-α-[äthyl]-benzhydrolderivate der allgemeinen Formel I in die entsprechenden freien Basen der allgemeinen Formel I und/oder in andere Säureadditionssalze überführt.

2. Verfahren nach Anspruch 1, dadurch gekennzeichnet, daß man als [ω-(Diäthylamino)-alkoxy]-α-[äthyl]-benzhydrolderivate solche, bei welchen das, beziehungsweise die, Halogenatom(e), für das, beziehungsweise die, $R_1$ und/oder $R_2$ stehen kann, beziehungsweise können, [ein] Chlor-, Fluor- und/oder Bromatom(e) ist, beziehungsweise sind, herstellt.

3. Verfahren nach Anspruch 1 oder 2, dadurch gekennzeichnet, daß man als [ω-(Diäthylamino)-alkoxy]-α-[äthyl]-benzhydrolderivate solche, bei welchen der, beziehungsweise die, Trihalogenmethylrest(e), für den, beziehungsweise die, $R_1$ und/oder $R_2$ stehen kann, beziehungsweise können, [ein] Trifluormethylrest(e) und/oder Trichlormethylrest(e), ist, beziehungsweise sind, herstellt.

4. Verfahren nach Anspruch 1 bis 3, dadurch gekennzeichnet, daß man als [ω-(Diäthylamino)-alkoxy]-α-[äthyl]-benzhydrolderivate solche, bei welchen der, beziehungsweise die, Alkyl- und/oder Alkoxyrest(e), für den, beziehungsweise die, $R_1$ und/oder $R_2$ stehen kann, beziehungsweise können, ein solcher, beziehungsweise solche, mit 1 oder 2, insbesondere 1, Kohlenstoffatom(en) ist, beziehungsweise sind, herstellt.

5. Verfahren nach Anspruch 1 bis 4, dadurch gekennzeichnet, daß man als [ω-(Diäthylamino)-alkoxy]-α-[äthyl]-benzhydrolderivate solche, bei welchen die Alkylreste, für die $R_3$ und $R_4$ stehen, solche mit 3 oder 4, insbesondere 3, Kohlenstoffatomen sind, herstellt.

6. Verfahren nach Anspruch 1 bis 5, dadurch gekennzeichnet, daß man als [ω-(Diäthylamino)-alkoxy]-α-[äthyl]-benzhydrolderivate solche, bei welchen n 2 bis 4, insbesondere 2 oder 3, ist, herstellt.

7. Verfahren nach Anspruch 1 bis 6, dadurch gekennzeichnet, daß man als [ω-(Diäthylamino)-alkoxy]-α-[äthyl]-benzhydrolderivate 3-Trifluormethyl-4'-[3-(diäthylamino)-propoxy]-α-äthyl-benzhydrol, 2-Methoxy-4'-[2-(diäthylamino)-äthoxy]-α-äthyl-benzhydrol, 4-Trifluormethyl-4'-[2-(diäthylamino)-äthoxy]-α-äthyl-benzhydrol, 3-Chlor-4'-[3-(diäthylamino)-propoxy]-α-äthyl-benzhydrol, 3-Chlor-4'-[2-(diäthylamino)-äthoxy]-α-äthyl-benzhydrol, 4-Fluor-4'-[3-(diäthylamino)-propoxy]-α-äthyl-benzhydrol, 2-Methoxy-4'-[3-(diäthylamino)-propoxy]-α-äthyl-benzhydrol, 2,5-Dimethyl-4'-[2-(diäthylamino)-äthoxy]-α-äthyl-benzhydrol, 4-Chlor-4'-[3-(diäthylamino)-propoxy]-α-äthyl-benzhydrol, 3-Trifluormethyl-4'-[2-(diäthylamino)-äthoxy]-α-äthyl-benzhydrol und 4-Chlor-4'-[2-(diäthylamino)-äthoxy]-α-äthyl-benzhydrol sowie ihre Salze herstellt.

**Claims** (for the Contracting States : BE, CH, DE, FR, GB, IT, LI, LU, NL, SE)

1. [ω-(diethylamino)-alkoxy]-α-[ethyl]-benzhydrol derivatives of the generic formula

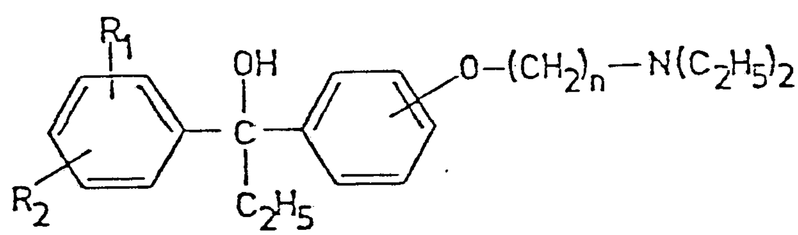

(I),

wherein

$R_1$ stands for a hydrogen, a halogen atom, a trihalogenmethyl radical or a straight or branched alkyl- or alkoxy radical having each 1 to 4 carbon atom(s),

$R_2$ means a halogen atom, a trihalogenmethyl radical or an alkyl- or alkoxy radical having each 1 to 4 carbon atom(s), and

n is an integer of 1 to 4,

and their acid addition salts and quaternary salts.

2. [ω-(diethylamino)-alkoxy]-α-[ethyl]-benzhydrol derivatives according to claim 1 characterized in that the halogen atom(s) for which $R_1$ and/or $R_2$ can stand, is (are) (a) chlorine-, fluorine- and/or bromine atom(s).

3. [ω-(diethylamino)-alkoxy]-α-[ethyl]-benzhydrol derivatives according to claim 1 or 2 characterized in that the trihalogenmethyl radical(s) for which $R_1$ and/or $R_2$ can stand, is (are) (a) trifluoromethyl radical(s) and/or trichloromethyl radical(s).

4. [ω-(diethylamino)-alkoxy]-α-[ethyl]-benzhydrol derivatives according the claim 1 to 3, characterized in that the alkyl- and/or alkoxy radical(s) for which $R_1$ and/or $R_2$ can stand, is (are) such one (those ones) having 1 or 2, especially 1 carbon atom(s).

5. [ω-(diethylamino)-alkoxy]-α-[ethyl]-benzohydrol derivatives according to claim 1 to 4, characterized in that the alkyl radicals for which $R_3$ and $R_4$ are standing are those having 3 or 4, especially 3, carbon atoms.

6. [ω-(diethylamino)-alkoxy]-α-[ethyl]-benzhydrol derivatives according to claim 1 to 5, characterized in that n is 2 to 4, especially 2 or 3.

7. The [ω-(diethylamino)-alkoxy]-α-[ethyl]-benzhydrol derivatives 3-trifluoromethyl-4′-[3-(diethylamino)-propoxy]-α-ethyl-benzhydrol, 2-methoxy-4′-[2-(diethylamino)-ethoxy]-α-ethyl-benzhydrol, 4-trifluoromethyl-4′-[2-(diethylamino)-ethoxy]-α-ethyl-benzhydrol, 3-chloro-4′-[3-(diethylamino)-propoxy]-α-ethyl-benzhydrol, 3-chloro-4′-[2-(diethylamino)-ethoxy]-α-ethylbenzhydrol, 4-fluoro-4′-[3-(diethylamino)-propoxy]-α-ethyl-benzhydrol, 2-methoxy-4′-[3-(diethylamino)-propoxy]-α-ethyl-benzhydrol, 2,5-dimethyl-4′-[2-(diethylamino)-ethoxy]-α-ethyl-benzhydrol, 4-chloro-4′-[3-(diethylamino)-propoxy]-α-ethyl-benzhydrol, 3-trifluoromethyl-4′-[2-(diethylamino)-ethoxy]-α-ethylbenzhydrol and 4-chloro-4′-[2-(diethylamino)-ethoxy]-α-ethyl-benzhydrol and their salts.

8. A process for preparing the compounds according to claim 1 to 7, characterized in that

a) propiophenones ω-(diethylamino)-alkoxy-substituted at the benzene ring of the generic formula

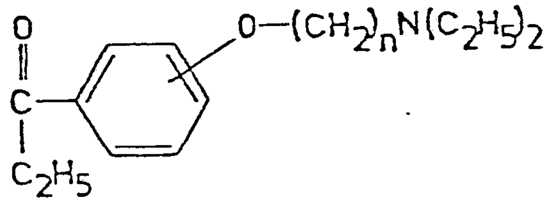

(II),

wherein n is as defined in claim 1 or 6 is reacted with organometallic compounds having a phenyl group of the generic formula

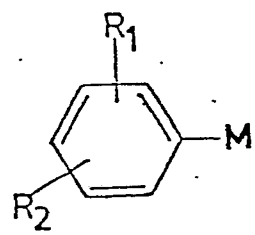

(III),

wherein

$R_1$ and $R_2$ are as defined in claims 1 to 4, and

M stands for an alkali metal atom, preferably lithium-, sodium, or potassium atom, or a radical of the formula MgX, X being a halogen atom, or

b) [ω-(halogen)-alkoxy]-α-[ethyl]-benzhydrol derivatives of the generic formula

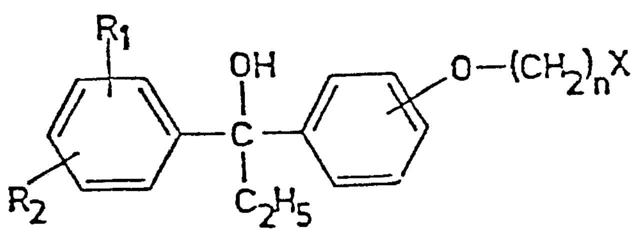

(IV),

wherein

$R_1$, $R_2$ and n are as defined in claims 1 to 4 or 6 and

X stands for a halogen atom,

are reacted with diethylamine or

c) [ω-(diethylamino)-alkoxy]-benzophenon derivatives of the generic formula

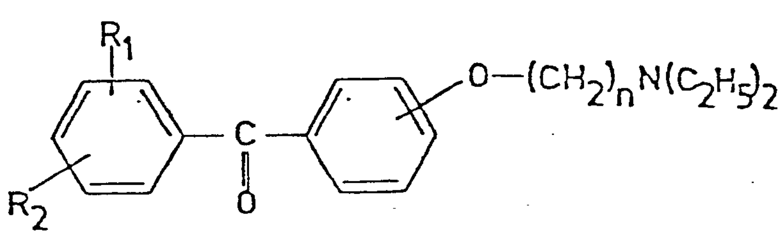

(V),

wherein $R_1$, $R_2$ and n are as defined in claims 1 to 4 or 6, are reacted with organometallic compounds having an ethyl group, especially ethylmagnesiumhalides or ethyllithium, or

d) propiones substituted at the benzene ring of the generic formula

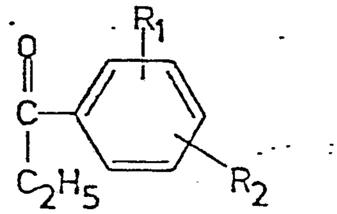

(VI),

wherein $R_1$ and $R_2$ are as defined in claims 1 to 4, are reacted with Grignard compounds having an {[ω-(diethylamino)-alkoxy]-phenyl}-group of the generic formula

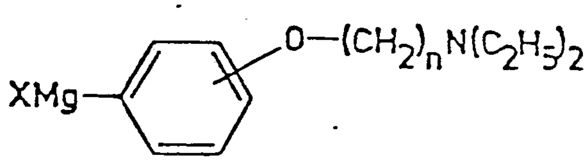

(VII),

wherein the meaning of

n is as defined in claim 1 or 6 and

X stands for a halogen atom, or

e) [hydroxy]-α-[ethyl]-benzhydrol derivatives of the generic formula

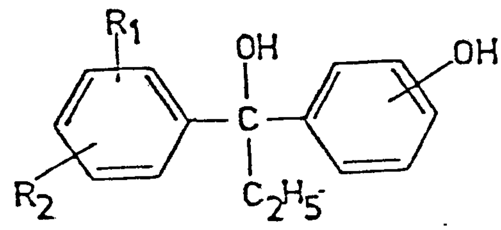

(VIII),

wherein $R_1$ and $R_2$ are as defined in claims 1 to 4, are reacted with tertiary amines of the generic formula

$$(C_2H_5)_2N—(CH_2)_n—X \qquad (IX),$$

wherein

X stands for an alkylsulfonyloxy- or arylsulfonyloxy radical or a halogen atom, and

n is as defined in claim 1 or 6, preferably in form of their alkali phenolates or quaternary ammonium phenolates or with their salts, preferably in the presence of acid binding agents, or

f) [ω-(diethylamino)-alkoxy]-α-[ethinyl]- and [ω-(diethylamino)-alkoxy]-α-[vinyl]-benzhydroles, respectively of the generic formula

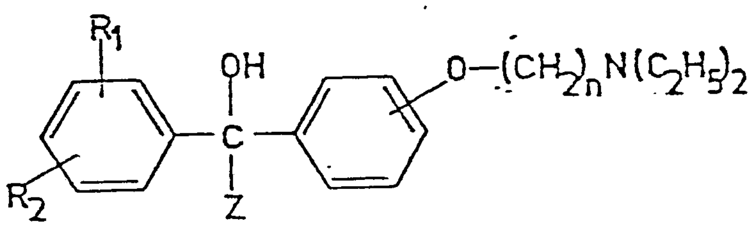

(X),

wherein

$R_1$, $R_2$ and n are as defined in claims 1 to 4 or 6, and

Z stands for an ethinyl- or vinyl radical

are reduced, whereafter optinally in a manner known per se the obtained [ω-(diethylamino)-alkoxy]-α-[ethyl]-benzhydrol derivatives of the generic formula I are converted to acid addition salts by the use of acids or to quaternary salts by use of quaternizing agents, or optionally the obtained acid addition salts of the [ω-(diethylamino)-alkoxy]-α-[ethyl]-benzhydrol derivatives of the generic formula I are converted to the corresponding free bases of the generic formula I and/or to different acid addition salts.

9. A pharmaceutical, characterized by a content of 1 or more compound(s) according to claim 1 to 7 as active ingredient(s), optionally together with 1 or more conventional pharmaceutical auxiliary agent(s).

**Claims** (for the Contracting State AT)

1. A process for preparing [ω-(diethylamino)-alkoxy]-α-[ethyl]-benzhydrol derivatives of the generic formula

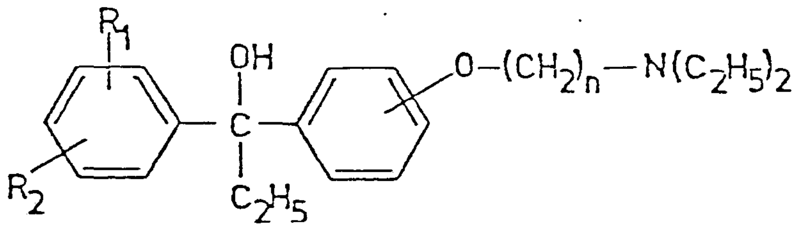

(I),

wherein

$R_1$ stands for a hydrogen atom, a halogen atom, a trihalogenmethyl radical or a straight or branched alkyl- or alkoxy radical having each 1 to 4 carbon atom(s),

$R_2$ means a halogen atom, a trihalogenmethyl radical or an alkyl- or alkoxy radical having each 1 to 4 carbon atom(s) and

n is an integer of 1 to 4,

and their acid addition salts and quaternary salts, characterized in that

a) propiophenones ω-(diethylamino)-alkoxy-substituted at the benzene ring of the generic formula

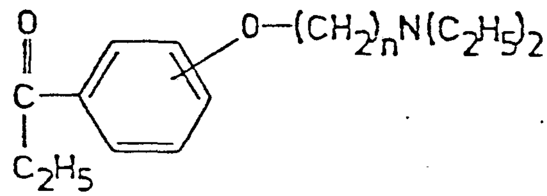

(II),

wherein n is as defined above is reacted with organometallic compounds having a phenyl group of the generic formula

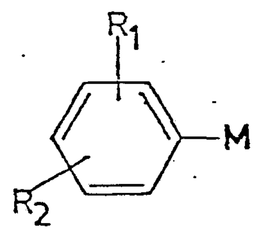

(III),

wherein

$R_1$ and $R_2$ are as defined above, and

M stands for an alkali metal atom, preferably lithium-, sodium-, or potassium atom, or a radical of the formula MgX, X being a halogen atom, or

b) [ω-(halogen)-alkoxy]-α-[ethyl]-benzhydrol derivatives of the generic formula

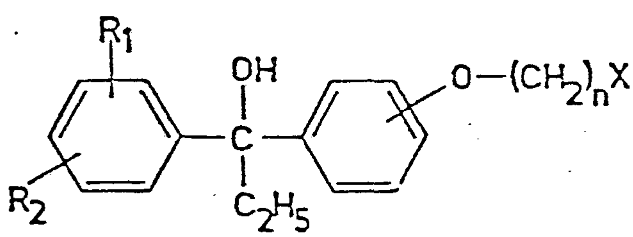

(IV),

wherein

$R_1$, $R_2$ and n are as defined above and

X stands for a halogen atom,

are reacted with diethylamine or

c) [ω-(diethylamino)-alkoxy]-benzophenon derivatives of the generic formula

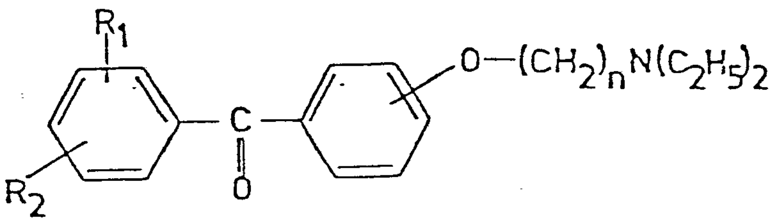

(V),

wherein $R_1$, $R_2$ and n are as defined above, are reacted with organometallic compounds having an ethyl group, especially ethylmagnesiumhalides or ethyllithium, or

d) propiones substituted at the benzene ring of the generic formula

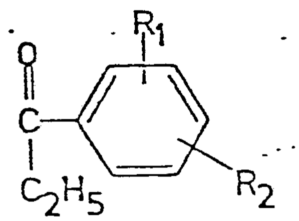

(VI),

wherein $R_1$ and $R_2$ are as defined above, are reacted with Grignard compounds having an {[ω-(diethylamino)-alkoxy]-phenyl}-group of the generic formula

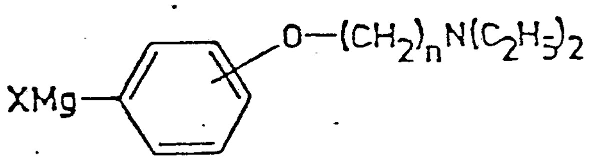

(VII),

wherein the meaning of

n is as defined above and

X stands for a halogen atom, or

e) [hydroxy]-α-[ethyl]-benzhydrol derivatives of the generic formula

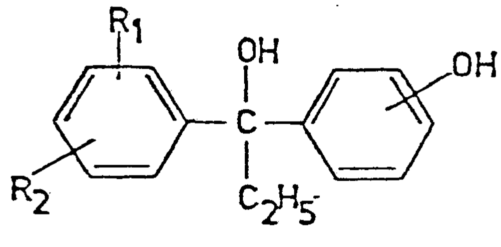

(VIII),

wherein $R_1$ and $R_2$ are as defined above, are reacted with tertiary amines of the generic formula

$$(C_2H_5)_2N-(CH_2)_n-X \quad \text{(IX)},$$

wherein

X stands for an alkylsulfonyloxy- or arylsulfonyloxy radical or a halogen atom, and

n is as defined above, preferably in form of their alkali phenolates or quaternary ammonium phenolates or with their salts, preferably in the presence of acid binding agents, or

f) [ω-(diethylamino)-alkoxy]-α-[ethinyl]- and [ω-(diethylamino)-alkoxy]-α-[vinyl]-benzhydroles, respectively of the generic formula

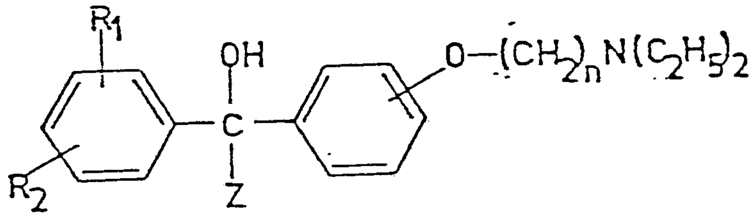

(X),

wherein

$R_1$, $R_2$ and n are as defined above, and

Z stands for an ethinyl- or vinyl radical

are reduced, whereafter optinally in a manner known per se the obtained [ω-(diethylamino)-alkoxy]-α-[ethyl]-benzhydrol derivatives of the generic formula I are converted to acid addition salts by the use of acids or to quaternary salts by use of quaternizing agents, or optionally the obtained acid addition salts of the [ω-(diethylamino)-alkoxy]-α-[ethyl]-benzhydrol derivatives of the generic formula I are converted to the corresponding free bases of the generic formula I and/or to different acid addition salts.

2. A process according to claim 1, characterized in that as [ω-(diethylamino)-alkoxy]-α-[ethyl]-benzhydrol derivatives those are produced in which the halogen atom(s) for which $R_1$ and/or $R_2$ can stand, is (are) (a) chlorine-, fluorine and/or bromine atom(s).

3. A process according to claim 1 or 2, characterized in that as [ω-(diethylamino)-alkoxy]-α-[ethyl]-benzhydrol derivatives those are prepared in which the trihalogenmethyl radical(s) for which $R_1$ and/or $R_2$ can stand, is (are) trifluoromethyl radical(s) and/or trichloromethyl radical(s).

4. A process according to claim 1 to 3, characterized in that as [ω-(diethylamino)-alkoxy]-α-[ethyl]-benzhydrol derivatives those are prepared in which the alkyl- and/or alkoxy radical(s) for which $R_1$ and/or $R_2$ can stand, is (are) such one (those ones) having 1 or 2, especially 1, carbon atom(s).

5. A process according to claim 1 to 4 characterized in that as [ω-(diethylamino)-alkoxy]-α-[ethyl]-benzhydrol derivatives those are prepared in which the alkyl radicals for which $R_1$ and $R_4$ are standing have 3 or 4, especially 3, carbon atoms.

6. A process according to claim 1 to 5, characterized in that as [ω-(diethylamino)-alkoxy]-α-[ethyl]-benzhydrol derivatives those are prepared in which n is 2 to 4, especially 2 or 3.

7. A process according to claim 1 to 6, characterized in that as [ω-(diethylamino)-alkoxy]-α-[ethyl]-benzhydrol derivatives 3-trifluoromethyl-4′-[3-(diethylamino)-propoxy]-α-ethyl-benzhydrol, 2-methoxy-4′-[2-(diethylamino)-ethoxy]-α-ethyl-benzhydrol, 4-trifluoromethyl-4′-[2-(diethylamino)-ethoxy]-α-ethyl-benzhydrol, 3-chloro-4′-[3-diethylamino)-propoxy]-α-ethyl-benzhydrol, 3-chloro-4′-[2-(diethylamino)-ethoxy]-α-ethyl-benzhydrol, 4-fluoro-4′-[3-(diethylamino)-propoxy]-α-ethyl-benzhydrol, 2-methoxy-4′-[3-(diethylamino)-propoxy]-α-ethyl-benzhydrol, 2,5-dimethyl-4′-[2-(diethylamino)-ethoxy]-α-ethylbenzhydrol, 4-chloro-4′-[3-(diethylamino)-propoxy]-α-ethyl-benzhydrol, 3-trifluoromethyl-4′-[2-diethylamino)-ethoxy]-α-ethyl-benzhydrol and 4-chloro-4′-[2-(diethylamino)-ethoxy]-α-ethyl-benzhydrol and their salts are prepared.

**Revendications** (pour les Etats contractants : BE, CH, DE, FR, GB, IT, LI, LU, NL, SE)

1. Dérivés de [ω-(diéthylamino)-alcoxy]-α-[éthyl]-benzhydrol répondant à la formule générale :

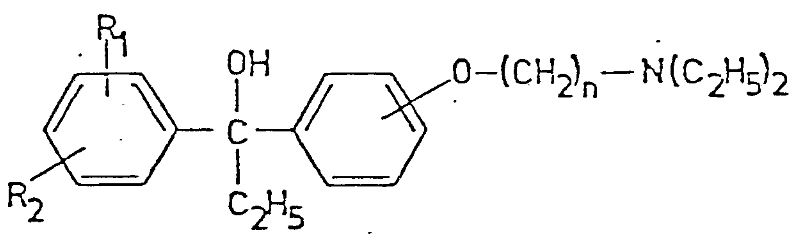

(I),

dans laquelle

$R_1$ représente un atome d'hydrogène, un atome d'halogène, un reste trihalométhyle ou bien un reste alcoyle ou alcoxy linéaire ou ramifié comportant, dans chaque cas, 1 à 4 atome(s) de carbone,

$R_2$ représente un atome d'halogène, un reste trihalométhyle ou bien un reste alcoyle ou alcoxy comportant, dans chaque cas, 1 à 4 atome(s) de carbone, et

n est un nombre entier de 1 à 4,

ainsi que leurs sels d'addition avec des acides et leurs sels quaternaires.

2. Dérivés de [ω-(diéthylamino)-alcoxy]-α-[éthyl]-benzhydrol selon la revendication 1, caractérisés en ce que l'atome et/ou les atomes d'halogène que peut ou peuvent représenter $R_1$ et/ou $R_2$, est (sont) un (des) atome(s) de chlore, de fluor et/ou de brome.

3. Dérivés de [ω-(diéthylamino)-alcoxy]-α-[éthyl]-benzhydrol selon la revendication 1 ou 2, caractérisés en ce que le et/ou les reste(s) trihalométhyle, que peut ou peuvent représenter $R_1$ et/ou $R_2$, est (sont), un (des) reste(s) trifluorométhyle et/ou trichlorométhyle.

4. Dérivés de [ω-(diéthylamino)-alcoxy]-α-[éthyl]-benzhydrol selon les revendications 1 à 3, caractérisés en ce que le et/ou les reste(s) alcoyle et/ou alcoxy, que peut ou peuvent représenter $R_1$ et/ou $R_2$, est (sont), un ou des reste(s) comportant un ou deux, notamment un atome(s) de carbone.

5. Dérivés de [ω-(diéthylamino)-alcoxy]-α-[éthyl]-benzhydrol selon les revendications 1 à 4, caractérisés en ce que les restes alcoyle que représentent $R_3$ et $R_4$ sont des restes comportant un ou quatre, notamment trois atomes de carbone.

6. Dérivés de [ω-(diéthylamino)-alcoxy]-α-[éthyl]-benzhydrol selon les revendications 1 à 5, caractérisés en ce que n est un entier de 2 à 4, notamment un entier égal à 2 ou 3.

7. Les dérivés de [ω-(diéthylamino)-alcoxy]-α-[éthyl]-benzhydrol suivants : les 3-trifluorométhyl-4'-[3-(diéthylamino)-propoxy]-α-éthyl-benzhydrol, 2-méthoxy-4'-[2-(diéthylamino)-éthoxy]-α-éthyl-benzhydrol, 4-trifluorométhyl-4'-[2-(diéthylamino)-éthoxy]-α-éthyl-benzhydrol, 3-chloro-4'-[3-(diéthylamino)-propoxy]-α-éthyl-benzhydrol, 3-chloro-4'-[2-(diéthylamino)-éthoxy]-α-éthyl-benzhydrol, 4-fluoro-4'-[3-(diéthylamino)-propoxy]-α-éthyl-benzhydrol, 2-méthoxy-4'-[3-(diéthylamino)-propoxy]-α-éthyl-benzhydrol, 2,5-diméthyl-4'-[2-(diéthylamino)-éthoxy]-α-éthyl-benzhydrol, 4-chloro-4'-[3-(diéthylamino)-propoxy]-α-éthyl-benzhydrol, 3-trifluorométhyl-4'-[2-(diéthylamino)-éthoxy]-α-éthyl-benzhydrol et 4-chloro-4'-[2-(diéthylamino)-éthoxy]-α-éthyl-benzhydrol, ainsi que leurs sels.

8. Procédé de préparation des composés selon les revendications 1 à 7, caractérisé en ce que

a) l'on fait réagir des phénones propioniques à substitution ω-(diéthylamino)-alcoxy sur le noyau benzénique, répondant à la formule générale :

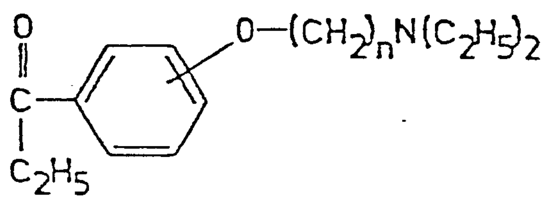

(II),

dans laquelle n a la signification de la revendication 1 ou 6, sur des composés organométalliques comportant un groupe phényle, répondant à la formule générale :

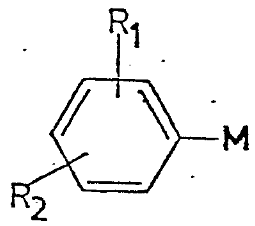

(III),

dans laquelle

$R_1$ et $R_2$ ont la même signification que dans les revendications 1 à 4, et

M représente un atome de métal alcalin, de préférence un atome de lithium, de sodium ou de potassium, ou un reste répondant à la formule MgX, X étant un atome d'halogène, ou bien

b) l'on fait réagir des dérivés de [ω-(halo)-alcoxy]-α-[éthyl]-benzhydrol répondant à la formule générale :

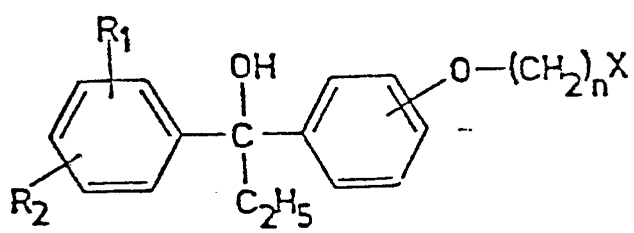

(IV),

dans laquelle

$R_1$, $R_2$ et n ont la même signification que dans les revendications 1 à 4 ou 6, et

X représente un atome d'halogène, sur de la diéthylamine, ou bien

c) l'on fait réagir des dérivés de [ω-(diéthylamino)-alcoxy]-benzophénone répondant à la formule générale :

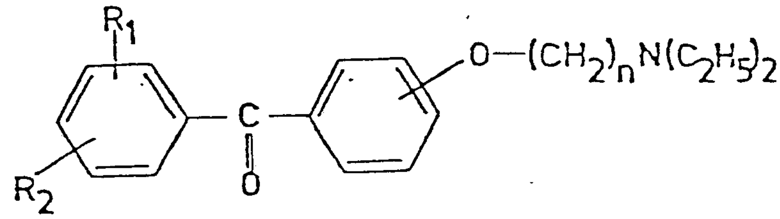

(V)

dans laquelle $R_1$, $R_2$ et n ont la même signification que dans les revendications 1 à 4 ou 6, sur des composés organométalliques comportant un groupe éthyle, notamment des halogénures d'éthyl-magnésium ou d'éthyl-lithium, ou bien

d) l'on fait réagir des phénones propioniques substituées sur leur noyau benzénique répondant à la formule générale :

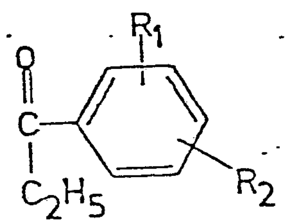

(VI)

dans laquelle $R_1$ et $R_2$ ont la même signification que dans les revendications 1 à 4, sur des composés de Grignard comportant un groupe {[ω-(diéthylamino)-alcoxy]-phényle} répondant à la formule générale :

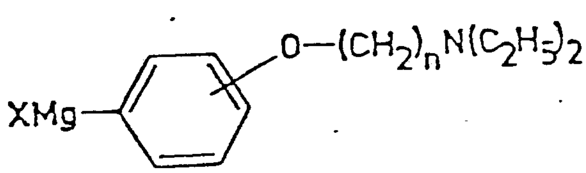

(VII)

dans laquelle

n a la même signification que dans la revendication 1 ou 6, et

X représente un atome d'halogène, ou bien

e) l'on fait réagir des dérivés de [hydroxy]-α-[éthyl]-benzhydrol répondant à la formule générale :

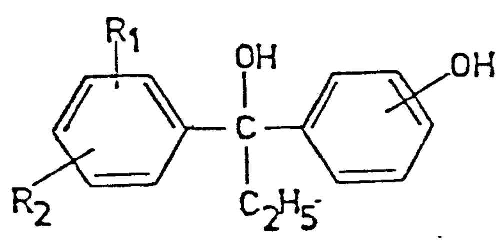

(VIII)

dans laquelle $R_1$ et $R_2$ ont la même signification que dans les revendications 1 à 4, de préférence sous la forme de leurs phénolates alcalins ou de leurs phénolates d'ammonium quaternaires, sur des amines tertiaires répondant à la formule générale :

$$(C_2H_5)_2N—(CH_2)_n—X \qquad (IX)$$

dans laquelle

X représente un reste alcoylsulfonyloxy ou arylsulfonyloxy, ou bien un atome d'halogène, et

n a la même signification que dans la revendication 1 ou 6, ou sur leurs sels, en présence d'agents de fixation des acides, ou bien

f) on réduit des [ω-(diéthylamino)-alcoxy]-α-[éthynyl] et/ou des [ω-(diéthylamino)-alcoxy]-α-[vinyl]-benzhydrols répondant à la formule générale :

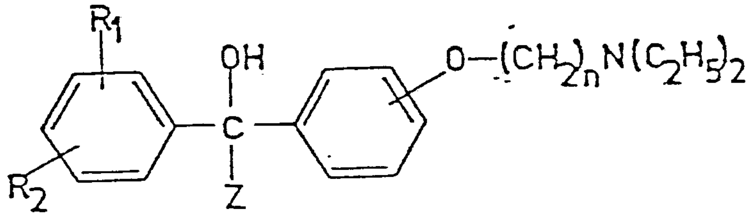

(X)

dans laquelle

$R_1$, $R_2$ et n ont la même signification que dans les revendications 1 à 4 ou 6, et

Z représente un reste éthylyle ou vinyle,

et l'on transforme alors éventuellement, de façon en soi connue, les dérivés de [ω-(diéthylamino)-alcoxy]-α-[éthyl]-benzhydrol répondant à la formule générale I obtenus avec des acides, en sels d'addition avec des acides, ou bien, avec des agents de transformation en dérivés quaternaires, en sels quaternaires, ou bien l'on transforme éventuellement les sels d'addition avec des acides des dérivés [ω-(diéthylamino)-alcoxy]-α-[éthyl]-benzhydrol répondant à la formule générale I obtenus en les bases libres correspondantes répondant à la formule générale I et/ou en d'autres sels d'addition avec des acides.

9. Médicament, caractérisé en ce qu'il renferme un ou plusieurs composé(s) selon les revendications 1 à 7, comme principe(s) actif(s), de façon appropriée en même temps qu'un ou plusieurs agent(s) de confection pharmaceutique(s) usuel(s).

**Revendications** (pour l'Etat contractant AT)

1. Procédé de préparation des dérivés de [ω-(diéthylamino)-alcoxy]-α-[éthyl]-benzhydrol répondant à la formule générale :

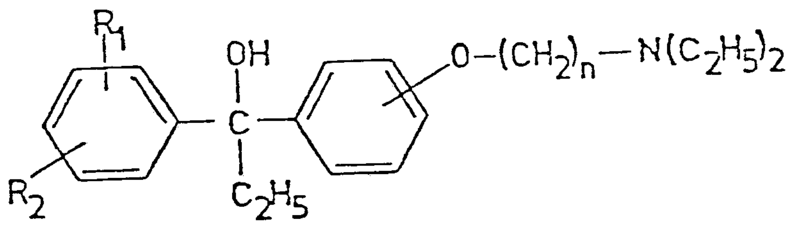

(I),

dans laquelle

$R_1$ représente un atome d'hydrogène, un atome d'halogène, un reste trihalométhyle ou bien un reste alcoyle ou alcoxy linéaire ou ramifié comportant, dans chaque cas, 1 à 4 atome(s) de carbone,

$R_2$ représente un atome d'halogène, un reste trihalométhyle ou bien un reste alcoyle ou alcoxy comportant, dans chaque cas, 1 à 4 atome(s) de carbone, et

n est un nombre entier de 1 à 4,

ainsi que leurs sels d'addition avec des acides et leurs sels quaternaires, caractérisé en ce que

a) l'on fait réagir des phénones propioniques à substitution ω-(diéthylamino)-alcoxy sur le noyau benzénique, répondant à la formule générale :

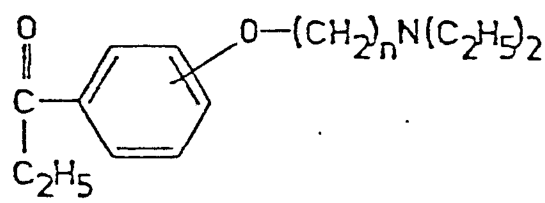

(II),

dans laquelle n a la signification de la revendication 1 ou 6, sur des composés organométalliques comportant un groupe phényle, répondant à la formule générale :

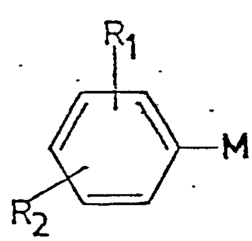

(III),

dans laquelle

$R_1$ et $R_2$ ont la même signification que dans les revendications 1 à 4, et

M représente un atome de métal alcalin, de préférence un atome de lithium, de sodium ou de potassium, ou un reste répondant à la formule MgX, X étant un atome d'halogène, ou bien

b) l'on fait réagir des dérivés de [ω-(halo)-alcoxy]-α-[éthyl]-benzhydrol répondant à la formule générale :

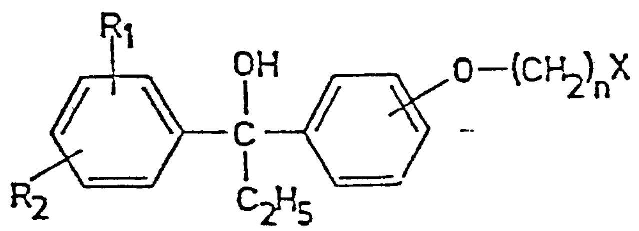

(IV),

dans laquelle

$R_1$, $R_2$ et n ont la même signification que dans les revendications 1 à 4 ou 6, et

X représente un atome d'halogène,

sur de la diéthylamine, ou bien

c) l'on fait réagir des dérivés de [ω-(diéthylamino-alcoxy]-benzophénone répondant à la formule générale :

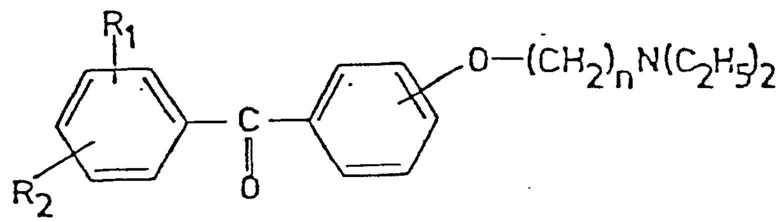

(V)

dans laquelle $R_1$, $R_2$ et n ont la même signification que dans les revendications 1 à 4 ou 6, sur des composés organométalliques comportant un groupe éthyle, notamment des halogénures d'éthyl-magnésium ou d'éthyl-lithium, ou bien

d) l'on fait réagir des phénones propioniques substituées sur leur noyau benzénique répondant à la formule générale :

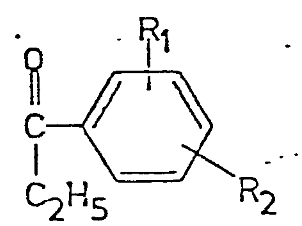

(VI)

dans laquelle $R_1$ et $R_2$ ont la même signification que dans les revendications 1 à 4, sur des composés de Grignard comportant un groupe {[ω-(diéthylamino)-alcoxy]-phényle} répondant à la formule générale :

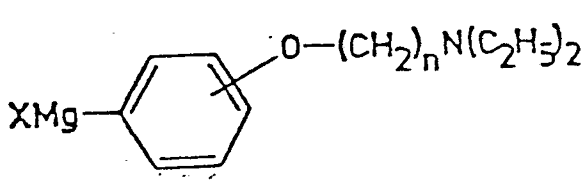

(VII)

dans laquelle)

n a la même signification que dans la revendication 1 ou 6, et

X représente un atome d'halogène, ou bien

e) l'on fait réagir des dérivés de [hydroxy]-α-[éthyl]-benzhydrol répondant à la formule générale :

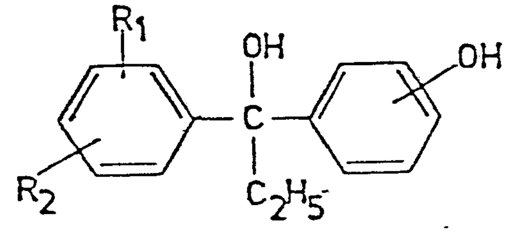

(VIII)

dans laquelle $R_1$ et $R_2$ ont la même signification que dans les revendications 1 à 4, de préférence sous la forme de leurs phénolates alcalins ou de leurs phénolates d'ammonium quaternaires, sur des amines tertiaires répondant à la formule générale :

$$(C_2H_5)_2N{-}(CH_2)_n{-}X \qquad \text{(IX)}$$

dans laquelle

X représente un reste alcoylsulfonyloxy ou arylsulfonyloxy, ou bien un atome d'halogène, et

n a la même signification que dans la revendication 1 ou 6, ou sur leurs sels, en présence d'agents de fixation des acides, ou bien

f) on réduit des [ω-(diéthylamino)-alcoxy]-α-[éthynyl] et/ou des [ω-(diéthylamino)-alcoxy]-α-[vinyl]-benzhydrols répondant à la formule générale :

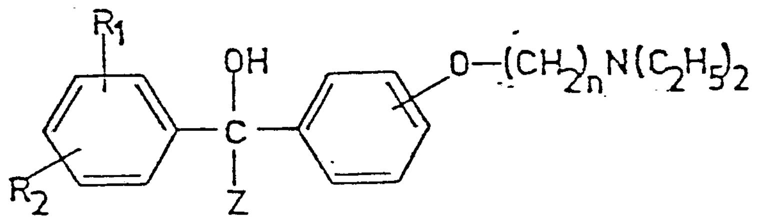

(X)

dans laquelle

$R_1$, $R_2$ et n ont la même signification que dans les revendications 1 à 4 ou 6, et

Z représente un reste éthylyle ou vinyle, et l'on transforme alors éventuellement, de façon en soi connue, les dérivés de [ω-(diéthylamino)-alcoxy]-α-[éthyl]-benzhydrol répondant à la formule générale I obtenus avec des acides, en sels d'addition avec des acides, ou bien, avec des agents de transformation en dérivés quaternaires, en sels quaternaires, ou bien l'on transforme éventuellement les sels d'addition avec des acides des dérivés [ω-(diéthylamino)-alcoxy]-α-[éthyl]-benzhydrol répondant à la formule générale I obtenus en les bases libres correspondantes répondant à la formule générale I et/ou en d'autres sels d'addition avec des acides.

2. Procédé de préparation des composés selon la revendication 1, caractérisé en ce que l'atome et/ou les atomes d'halogène que peut ou peuvent représenter $R_1$ et/ou $R_2$ dans la formule générale I est (sont) un (des) atome(s) de chlore, de fluor et/ou de brome.

3. Procédé des préparations des composés selon les revendications 1 à 2, caractérisé en ce que le et/ou les reste(s) trihalométhyle, que peut ou peuvent représenter $R_1$ et/ou $R_2$ dans la formule générale I est (sont), un (des) reste(s) trifluorométhyle et/ou trichlorométhyle.

4. Procédé de préparation des composés selon les revendications 1 à 3, caractérisé en ce que le et/ou les reste(s) alcoyle et/ou alcoxy, que peut ou peuvent représenter $R_1$ et/ou $R_2$ dans la formule générale I, est (sont), un ou des reste(s) comportant un ou deux, notamment un atome(s) des carbones.

5. Procédé de préparation des composés selon les revendications 1 à 4, caractérisé en ce que les restes alcoyle que représentent $R_3$ et $R_4$ dans la formule générale I sont des restes comportant un ou quatre, notamment trois atomes de carbone.

6. Procédé de préparation des composés selon les revendications 1 à 5, caractérisé en ce que n dans la formule générale I est un entier de 2 à 4, notamment un entier égal à 2 ou 3.

7. Procédé de préparation des composés selon les revendications 1 à 6, caractérisé en ce que 3-trifluorométhyl-4'-[3-(diéthylamino)-propoxy]-α-éthyl-benzhydrol, 2-méthoxy-4'-[2-(diéthylamino)-éthoxy]-α-éthyl-benzhydrol, 4-trifluorométhyl-4'-[2-(diéthylamino)-éthoxy]-α-éthyl-benzhydrol, 3-chloro-4'-[3-(diéthylamino)-propoxy]-α-éthyl-benzhydrol, 3-chloro-4'-[2-(diéthylamino)-éthoxy]-α-éthyl-benzhydrol, 4-fluoro-4'-[3-(diéthylamino)-propoxy]-α-éthyl-benzhydrol, 2-méthoxy-4'-[3-(diéthylamino)-propoxy]-α-éthyl-benzhydrol, 2,5-diméthyl-4'-[2-(diéthylamino)-éthoxy]-α-éthyl-benzhydrol, 4-chloro-4'-[3-(diéthylamino)-propoxy]-α-éthyl-benzhydrol, 3-trifluorométhyl-4'-[2-(diéthylamino)-éthoxy]-α-éthyl-benzhydrol et 4-chloro-4'-[2-(diéthylamino)-éthoxy]-α-éthyl-benzhydrol, ainsi que leurs sels sont préparés.